(19) Europäisches Patentamt
European Patent Office
Office européen des brevets

(11) Numéro de publication: **0 272 982 B1**

(12) # FASCICULE DE BREVET EUROPEEN

(45) Date de publication de fascicule du brevet: **17.04.91**

(51) Int. Cl.5: **C07C 233/77**, C07D 295/12, C07D 333/60, C07D 333/24, A61K 31/165, A61K 31/38, A61K 31/395

(21) Numéro de dépôt: **87402891.3**

(22) Date de dépôt: **17.12.87**

(54) **Nouveaux dérivés de l'indane, leur procédé de préparation et les nouveaux intermédiaires ainsi obtenus, leur application comme médicaments et les compositions les renfermant.**

(30) Priorité: **23.12.86 FR 8618026**

(43) Date de publication de la demande: **29.06.88 Bulletin 88/26**

(45) Mention de la délivrance du brevet: **17.04.91 Bulletin 91/16**

(84) Etats contractants désignés: **AT BE CH DE ES FR GB GR IT LI LU NL SE**

(56) Documents cités:
**EP-A- 0 108 602**
**EP-A- 0 147 085**
**EP-A- 0 260 555**

(73) Titulaire: **ROUSSEL-UCLAF**
**35, boulevard des Invalides**
**F-75007 Paris(FR)**

(72) Inventeur: **Clémence, François**
**2, rue Turgot**
**F-75009 Paris(FR)**
Inventeur: **Le Martret, Odile**
**42, Avenue de Versailles**
**F-75016 Paris(FR)**
Inventeur: **Fortin, Michel**
**12, Passage Cottin**
**F-75018 Paris(FR)**
Inventeur: **Delevallée, Françoise**
**48-50, avenue de la Dame Blanche**
**F-94120 Fontenay-sous-Bois(FR)**

(74) Mandataire: **Bourgouin, André et al**
**Département des Brevets ROUSSEL UCLAF**
**111, route de Noisy B.P. no 9**
**F-93230 Romainville(FR)**

**EP 0 272 982 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

L'invention concerne de nouveaux dérivés de l'indane, leur procédé de préparation et les nouveaux intermédiaires ainsi obtenus, leur application à titre de médicaments et les compositions pharmaceutiques les renfermant.

L'invention a pour objet les composés de formule (I) :

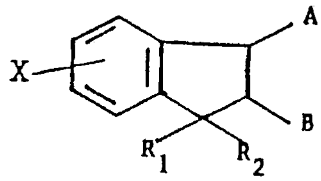

(I)

dans laquelle X représente un atome d'hydrogène, un atome d'halogène, un radical alkyle renfermant de 1 à 5 atomes de carbone, un radical alkoxy renfermant de 1 à 5 atomes de carbone, $R_1$ et $R_2$, identiques ou différents, représentent un atome d'hydrogène ou un radical alkyle renfermant de 1 à 5 atomes de carbone et A et B sont tels que l'un des substituants A ou B est choisi dans :

- le groupe de formule :

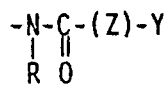

R étant un atome d'hydrogène ou un radical alkyle renfermant de 1 à 5 atomes de carbone, Z une chaîne alkylène linéaire $-(CH_2)_n-$, n étant un nombre entier pouvant varier entre 0 et 5 ou une chaîne alkylène ramifiée renfermant de 2 à 8 atomes de carbone, Y représentant un radical naphtyle ou indényle ou un radical hétéromonocyclique renfermant 5 ou 6 chaînons ou hétérobicyclique, tous les radicaux mentionnés précédemment étant substitués éventuellement par un ou plusieurs radicaux choisis parmi les radicaux alkyle ou alkoxy renfermant de 1 à 5 atomes de carbone, halogène, hydroxyle, trifluorométhyle, nitro, amino, monoalkyl- ou dialkylamino ou phényle lui-même substitué éventuellement par un ou plusieurs radicaux choisis parmi les radicaux alkyle ou alkoxy renfermant de 1 à 4 atomes de carbone et halogène et l'autre des substituants A ou B est choisi dans

- le groupe de formule :

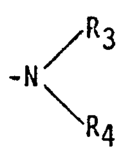

$R_3$ et $R_4$, identiques ou différents, représentant un atome d'hydrogène ou un radical alkyle renfermant de 1 à 5 atomes de carbone ou $R_3$ et $R_4$ forment ensemble avec l'atome d'azote auquel ils sont liés un hétérocycle à 5 ou 6 chaînons comportant éventuellement un autre hétéroatome tel que l'oxygène ou l'azote ou le soufre, lesdits composés de formule (I) pouvant être dans toutes les formes énantiomères et diastéréoisomères possibles et sous forme de sels d'addition avec les acides.

Lorsque X, $R_1$, $R_2$, R, $R_3$ et $R_4$ représentent un radical alkyle, il s'agit de préférence des radicaux méthyle, éthyle, n-propyle ou isopropyle mais ces substituants peuvent également représenter un radical n-butyle, isobutyle, n-pentyle.

Lorsque X est un atome d'halogène, il s'agit de préférence de l'atome de chlore, mais X peut aussi représenter un atome de fluor, de brome ou d'iode.

Lorsque X est un radical alkoxy, il s'agit de préférence d'un radical méthoxy ou éthoxy, mais X peut aussi représenter un radical propoxy, isopropoxy, butoxy linéaire, secondaire ou tertiaire.

Lorsque Z représente un groupe $-(CH_2)_n$, n est de préférence égal à 0 ou à 1.

Lorsque Z est une chaîne alkylène ramifiée, il s'agit de préférence d'une chaîne substituée par un ou plusieurs radicaux méthyle ou éthyle. Il s'agit par exemple de la chaîne 1,1-éthanediyl ; méthyl-1 éthanediyl-1,2 ; méthyl-1 ou 2 propanediyl-1,2 ; éthyl-1 éthanediyl-1,2.

Lorsque Y représente un radical hétéromono cyclique à 5 ou 6 chaînons, il s'agit de préférence du radical thiazolyle, pyridinyle, oxazolyle, isoxazolyle, imidazolyle ou thiényle.

Lorsque Y représente un radical hétérobicyclique, il s'agit par exemple d'un radical indolyle, quinolyle, benzofurannyle, benzo [b] thiényle, benzimidazolyle, benzoxazolyle ou benzothiazolyle.

Lorsque Y représente un radical naphtyle, indényle, hétéromonocyclique ou hétérobicyclique substitué, il s'agit de préférence d'untel radical substitué par un ou deux radicaux choisis parmi les radicaux méthyle ou éthyle, méthoxy ou éthoxy, un atome de chlore, brome ou fluor, trifluorométhyle, nitro ou phényle.

Lorsque les substituants sont des radicaux monoalkyl- ou dialkylamino, il s'agit de préférence des radicaux monalkyl- ou dialkylamino dans lesquels les radicaux alkyles sont les radicaux méthyle ou éthyle.

Le radical phényle est lui-même éventuellement substitué de préférence par un ou deux radicaux choisis parmi les radicaux méthyle, éthyle, méthoxy, éthoxy, un ou plusieurs atomes de chlore, brome ou fluor.

$R_3$ et $R_4$ forment de préférence avec l'atome d'azote auquel ils sont liés un radical pyrrolidinyle, pipérazinyle, pipéridinyle, morpholinyle.

Les sels d'addition avec les acides minéraux ou organiques peuvent être par exemple, les sels formés avec les acides chlorhydrique, bromhydrique, nitrique, sulfurique, phosphorique, acétique, propionique, formique, benzoïque, maléique, fumarique, succinique, tartrique, citrique, oxalique, glyoxylique, aspartique, alcanesulfoniques tels que l'acide méthane sulfonique et arylsulfoniques, tels que l'acide benzène sulfonique.

L'invention concerne notamment les composés de formule (I) caractérisés en ce que les groupements A et B sont en configuration trans ainsi que leurs sels d'addition avec les acides.

L'invention concerne plus particulièrement les composés de formule (I) caractérisés en ce que dans le groupe

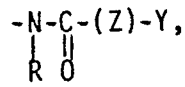

R représente un atome d'hydrogène, un radical méthyle ou éthyle, Z représente un groupe

$-CH_2-$ ou 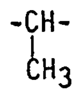,

Y représente un radical naphtyle, indényle, benzo [b] thiényle, indolyle, benzofurannyle, quinolyle, benzimidazolyle, benzoxazolyle, benzothiazolyle, thiényle éventuellement substitué par un radical phényle, ainsi que leurs sels d'addition avec les acides.

Elle a plus particulièrement pour objet les composés de formule (I) caractérisé en ce que X, $R_1$ et $R_2$ représentent un atome d'hydrogène et en ce que dans le groupe :

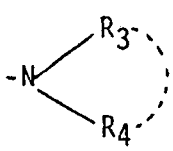,

$R_3$ et $R_4$ forment avec l'atome d'azote auquel ils sont liés l'hétérocycle pyrrolidine, ainsi que leurs sels d'addition avec les acides et tout particulièrement :

- le (trans) (±) N-[2,3-dihydro 2-(1-pyrrolidinyl 1H-inden 1-yl] N-méthyl 4-benzo [b] thiophène acétamide et ses sels avec les acides et le (trans) (±) N-[2,3-dihydro 2-(1-pyrrolidinyl) 1H-inden 1-yl] N-méthyl 1-naphtalène acétamide et ses sels avec les acides.

L'invention concerne aussi un procédé de préparation des produits de formule (I) dont les groupements A et B sont en configuration trans, caractérisé en ce que l'on condense le produit de formule (II) :

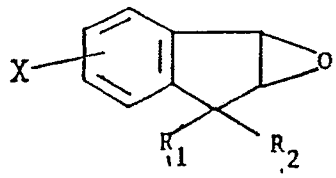

(II)

X, $R_1$ et $R_2$ ayant les significations données précédemment,
- soit avec une amine de formule (III) :

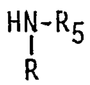

(III)

R ayant la signification donnée précédemment et $R_5$ étant un groupement protecteur de la fonction amine et notamment un groupement benzyle, pour obtenir un produit de formule (IV) :

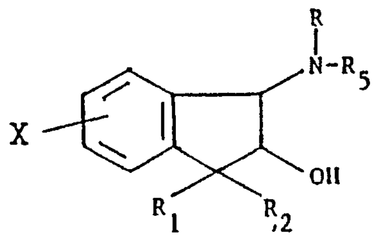

(IV)

dont on active la fonction hydroxyle et que l'on condense avec une amine de formule (V) :

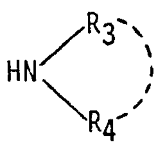

(V)

dans laquelle $R_3$ et $R_4$ ont les significations indiquées précédemment pour obtenir un produit de formule (VI) :

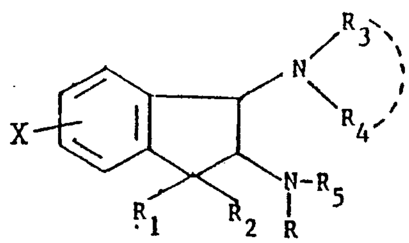

(VI)

dont on élimine le groupement protecteur $R_5$ de la fonction amine pour obtenir un produit de formule (VII) :

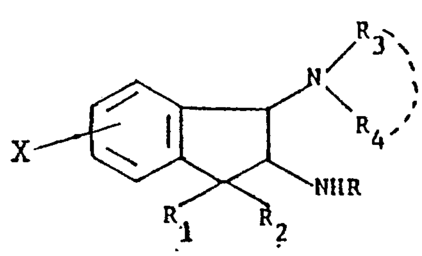

(VII)

que l'on traite avec un acide de formule (VIII) ou un dérivé fonctionnel de cet acide :

Y-(Z)-COOH (VIII)

dans laquelle Y et Z, ont les significations précédentes pour obtenir un produit de formule (I) dans laquelle A représente le

groupe- 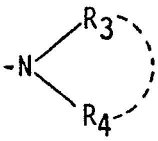 et B le groupe 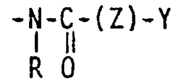

- soit avec une amine de formule (V) :

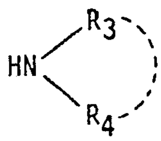

(V)

pour obtenir un produit de formule (IX) :

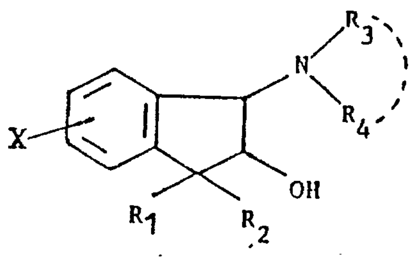

(IX)

dont on active la fonction hydroxyle et que l'on traite avec une amine de formule (X) :

$NH_2R$ (X)

dans laquelle R a la signification précédente pour obtenir un produit de formule (XI) :

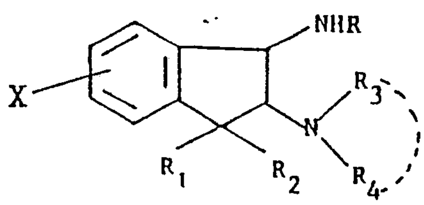

(XI)

que l'on condense avec un acide de formule (VIII) ou un dérivé fonctionnel de cet acide :

Y-(Z)-COOH (VIII)

dans laquelle Z et Y ont les significations précédentes pour obtenir un produit de formule (I) dans laquelle A représente :

le groupe 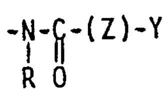 et B le groupe 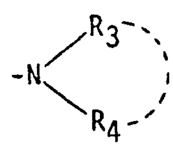 ,

lesdits composés de formule (I) pouvant être dédoublés pour obtenir les formes optiquement actives et que l'on traite, si désiré, avec un acide minéral ou organique, pour obtenir un sel.

Dans les conditions préférentielles du procédé de l'invention :

- Le chlorure de méthane sulfonyle est utilisé pour activer la fonction hydroxyle des produits de formule (IV) et des produits de formule (IX).
- Dans les produits de formule (VI), le groupement de protection $R_5$ est un radical benzyle qui peut être éliminé par hydrogénation catalytique. Le catalyseur que l'on utilise est de préférence le palladium.
- L'activation de la fonction hydroxyle des composés de formule (VIII) pour réaliser la condensation avec le composé de formule (VII) ou (XI) s'effectue en présence de carbonyldiimidazole. On peut également activer les acides de formule (VIII) sous la forme d'un chlorure d'acide ou d'anhydride mixte.
- Les produits de formule (I) peuvent être dédoublés par les méthodes usuelles.

Les produits de formule (I) pour lesquels les groupements A et B sont en configuration cis peuvent être préparés notamment selon le schéma suivant :

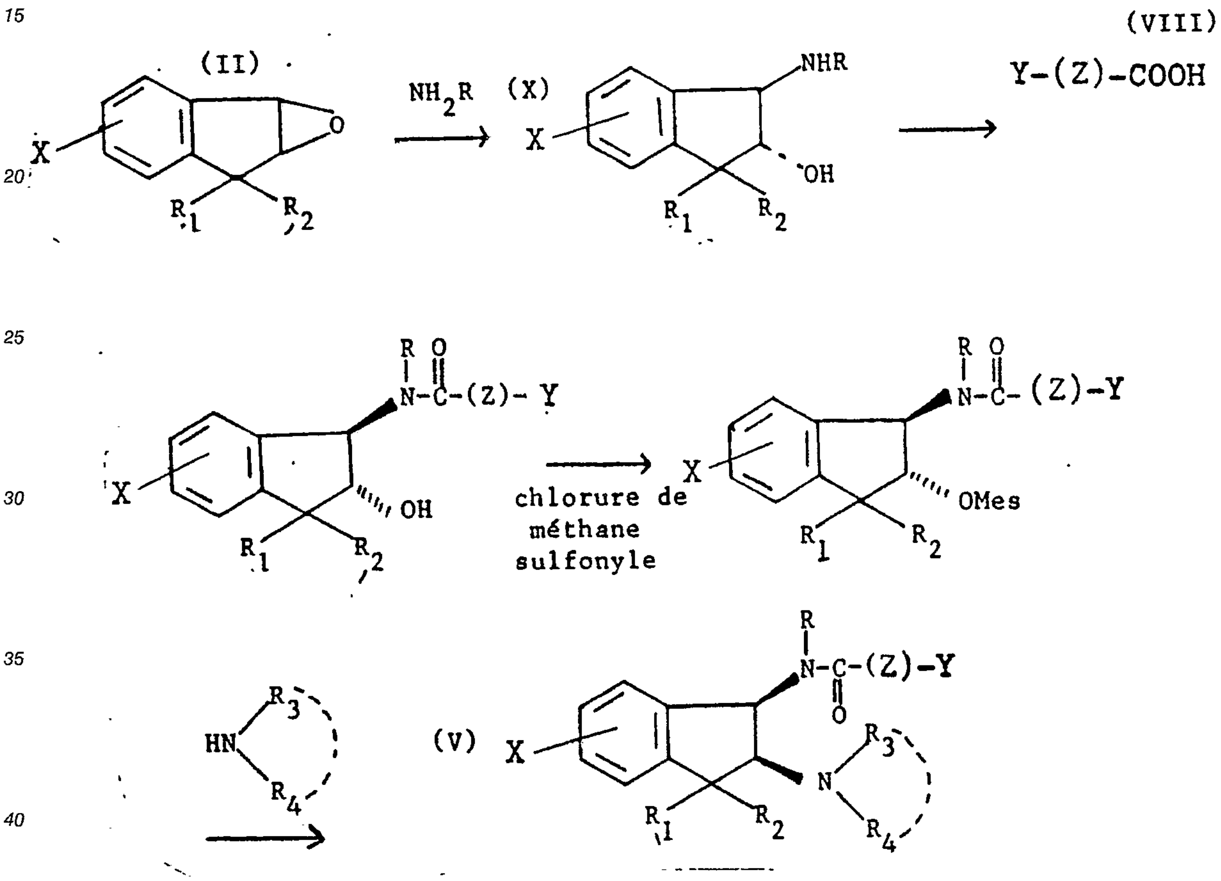


Les composés de formule (I) tels que définis ci-dessus ainsi que leurs sels d'addition avec les acides présentent d'intéressantes propriétés pharmacologiques. Ils présentent en particulier une forte affinité pour les récepteurs opiacés et notamment pour les récepteurs K et sont doués de propriétés analgésiques centrales. Les produits présentent, en outre, une activité diurétique, des propriétés anti-arythmiques, anti-ischémiques cérébrales et hypotensives.

Ces propriétés justifient l'utilisation des dérivés de l'indane ainsi que de leurs sels pharmaceutiquement acceptables à titre de médicaments.

La présente demande a ainsi également pour objet l'application à titre de médicaments, des dérivés de l'indane de formule (I) ainsi que de leurs sels d'addition avec les acides pharmaceutiquement acceptables.

Parmi les médicaments, objet de l'invention, on retient de préférence les médicaments caractérisés en ce qu'ils sont constitués par les composés de formule (I) dans laquelle les groupements A et B sont en configuration trans ainsi que leurs sels d'addition avec les acides pharmaceutiquement e acceptables.

Parmi ceux-ci on retient notamment ceux répondant à la formule (I) contenant un groupe

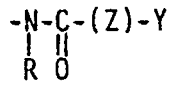

dans lequel R représente un atome d'hydrogène, un radical méthyle ou éthyle, Z représente un groupe $-CH_2-$ou

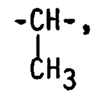

Y représente un radical naphtyle, indényle, benzo [b] thiényle, indolyle, benzofurannyle, quinolyle, benzimidazolyle, benzoxazolyle, benzothiazolyle, thiényle éventuellement substitué par un radical phényle, ainsi que leurs sels d'addition avec les acides pharmaceutiquement acceptables.

Parmi ceux-ci, on retient particulièrement ceux répondant à la formule (I) dans laquelle X, $R_1$ et $R_2$ représentent un atome d'hydrogène et dans le groupe :

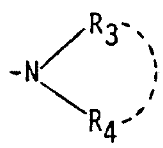

$R_3$ et $R_4$ forment avec l'atome d'azote auquel ils sont liés l'hétérocycle pyrrolidine, ainsi que leurs sels d'addition avec les acides pharmaceutiquement acceptables.

Parmi ces derniers, on retient tout particulièrement :

- le (trans) (±) N-[2,3-dihydro 2-(1-pyrrolidinyl 1H-inden 1-yl] N-méthyl 4-benzo [b] thiophène acétamide,
- le trans (±) N-[2,3-dihydro 2-(1-pyrrolidinyl 1H-inden 1-yl] N-méthyl 1-naphtalène acétamide et leurs sels avec les acides pharmaceutiquement acceptables.

Les médicaments, objet de l'invention, permettent notamment de soulager une douleur quelle qu'en soit l'origine, par exemple une douleur de nature musculaire, articulaire ou nerveuse.

Ils peuvent être aussi utilisés dans le traitement des douleurs dentaires, des migraines, du zona dans le traitement des douleurs intenses, en particulier rebelles aux antalgiques périphériques, par exemple au cours du processus néoplasiques, dans le traitement des pancréatites, coliques néphrétiques ou biliaires, dans le traitement des douleurs post-opératoires et post-traumatiques.

La posologie varie notamment en fonction de la voie d'administration, de l'affection traitée et du sujet en cause.

Par exemple, chez l'adulte, elle peut varier entre 20 et 400 mg de principe actif par jour, par voie orale, et entre 5 et 100 mg par jour, par voie parentérale.

Les médicaments, objet de l'invention, trouvent aussi leur emploi dans le traitement des arythmies.

La dose usuelle, dans ce traitement, variable selon le dérivé utilisé, le sujet et l'affection en cause, peut être par exemple de 50 mg à 1 g par jour.

Par voie orale, le principe actif peut être administré à la dose quotidienne de 200 mg à 800 mg, par exemple pour le traitement des arythmies ventriculaires, supraventriculaires et jonctionnelles , soit environ de 3 mg à 12 mg par kilogramme de poids corporel.

Les médicaments, objet de l'invention, peuvent aussi être utilisés dans le traitement des syndromes oedémateux, de l'insuffisance cardiaque, de certaines obésités, des cirrhoses, dans le traitement des oedèmes sévères et réfractaires, en particulier ceux de l'insuffisance cardiaque congestive et dans le traitement au long cours de l'hypertension artérielle.

La dose quotidienne de principe actif est variable. Elle peut être par exemple de 60 à 100 mg/jour par voie orale.

L'invention s'étend aux compositions pharmaceutiques renfermant comme principe actif les médicaments définis ci-dessus.

Ces compositions pharmaceutiques peuvent être administrées par voie buccale, rectale, par voie parentérale ou par voie locale en application topique sur la peau et les muqueuses.

Ces compositions peuvent être solides ou liquides et se présenter sous les formes pharmaceutiques couramment utilisées en médicine humaine comme par exemple, les comprimés simples ou dragéifiés, les gélules, les granulés, les suppositoires, les préparations injectables, les pommades, les crèmes, les gels et

les préparations en aérosols ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émusifiants, les conservateurs.

La posologie varie notamment en fonction de la voie d'administration de l'affection traitée et du sujet en cause.

Par ailleurs, les produits de départ de formule (II) pour lesquels $R_1$ et $R_2$ sont des atomes d'hydrogène ou des radicaux alcoyles renfermant de 1 à 5 atomes de carbone sont préparés par oxydation de l'indène correspondant.

Les composés de formule (IV), (VI), (VII), (IX) et (XI), dans lesquelles X ne représente pas un atome d'hydrogène, sont des produits chimiques nouveaux.

L'invention a donc pour objet ces produits à titre de produits industriels nouveaux, notamment à titre de produits intermédiaires nécessaires à la mise en oeuvre du procédé de l'invention.

Les exemples donnés ci-après illustrent l'invention sans toutefois la limiter.

**Exemple 1 : le (trans) (±) N-[2,3-dihydro 2-(1-pyrrolidinyl) 1H-inden 1-yl] N-méthyl 4-benzo [b] thiophène acétamide et son chlorhydrate.**

**Stade A:** Trans (±) 2,3-dihydro 1-(1-pyrrolidinyl) 1H-inden 2-ol.

On ajoute lentement 10,8 cm3 de pyrrolidine dans 6,75 g de 2,3-époxy indane (décrit par Mousseron et coll., Bulletin de la Société Chimique de France, 1946, p. 629-630) dans 10,8 cm3 d'eau déminéralisée. La température augmente à 65° C et on agite la solution 1 heure 30 minutes à cette température. On ajoute 20 cm3 d'eau déminéralisée en fin de réaction, distille l'excès de pyrrolidine sous pression réduite, obtient une phase huileuse et une phase aqueuse, sature au chlorure de sodium à 20° C, ajoute 1 cm3 de lessive de soude à 32% et extrait à l'éther. On sèche, concentre par distillation sous pression réduite, purifie l'huile obtenue par chromatographie sur silice (éluant : acétate d'éthyle à 5% de triéthylamine) et obtient 8,81 g de produit attendu.

**Stade B:** [Trans (±)] 2,3-dihydro N-méthyl 2-(1-pyrrolidinyl) 1H-indèn 1-amine.

On refroidit à -20° C un mélange renfermant 8,71 g de produit obtenu au stade A, 87 cm3 de chlorure de méthylène et 13,8 cm3 de triéthylamine et y introduit à cette température une solution renfermant 6,6 cm3 de chlorure de méthane sulfonyle et 8,7 cm3 de chlorure de méthylène. ON agite 20 minutes à -20° C, laisse revenir à 0° C, lave à l'eau glacée, extrait les lavages par du chlorure de méthylène. On sèche les phases organiques, concentre à sec sous pression réduite et obtient 13,36 g d'une résine correspondant au Trans (±) méthane sulfonate de 2,3-dihydro 1-(1-pyrrolidinyl) 1H-indèn-2-ol. Le résidu est traité dans un autoclave par une solution aqueuse à 35-40% de monométhylamine. On chauffe à 80° C durant 20 heures (pression stabilisée à 3 bars). On refroidit à 20° C, reprend par 100 cm3 d'éther, sature au chlorure de sodium, décante, lave la phase organique à l'eau salée saturée. On sèche, traite au charbon actif, filtre, rince, concentre à sec sous pression réduite. On obtient 6,98 g d'une résine que l'on purifie par chromatographie sur silice (éluant : acétate d'éthyle-méthanol-triéthylamine 85-10-5). On obtient 3,71 g de produit attendu.

**Stade C:** le (trans) (±) N-[2,3-dihydro 2-[1-pyrrolidinyl 1H-inden 1-yl] N-méthyl 4-benzo [b] thiophène acétamide et son chlorhydrate.

On agite pendant 1 heure : 0,5 g-d'acide 4-thianaphtène acétique, 0,422 g de carbonyldiimidazole et 20 cm3 de tétrahydrofuranne, puis ajoute lentement une solution de 0,432 g de produit obtenu au stade B dans 5 cm3 de tétrahydrofuranne. On agite pendant 3 heures 30 minutes, distille le tétrahydrofuranne sous pression réduite, reprend le résidu par 100 cm3 d'éther, lave par une solution saturée de bicarbonate de sodium, puis par de l'eau salée saturée, sèche, concentre à sec sous pression réduite et obtient 0,61 g de produit attendu (huile) sous forme de base.

On dissout à 50° C 0,405 g le produit obtenu précédemment dans 4 cm3 d'éthanol 99°, ajoute à chaud 0,5 cm3 d'une solution éthanolique d'acide chlorhydrique anhydre (titre = 5,75 N). On filtre aussitôt à chaud, rince à l'éthanol à 50° C, amorce la cristallisation à 30° C, laisse cristalliser au repos à 20° C durant 2 heures. On essore, rince à l'éthanol et à l'éther, sèche sous pression reduite et obtient 0,150 g du chlorhydrate attendu.

F = 192° C.

**Exemple 2 : Trans (±) N-[2,3-dihydro 2-(1-pyrrolidinyl) 1H-inden-1-yl] N-méthyl 5-phényl 2-thiophène acétamide et son chlorhydrate.**

On opère comme au stade C de l'exemple 1 à partir de 0,432 g de produit préparé comme au stade B, 0,567 g d'acide 5-phényl 2-thiophène acétique et 0,422 g de carbonyldiimidazole. On obtient 0,571 g de base et 0,457 g de chlorhydrate attendu. F ≃ 249° C.

Analyse : $C_{26}H_{28}N_2OS$, HCl : 453,05

| | | | | | |
|---|---|---|---|---|---|
| Calculé : | C% 68,93 | H% 6,45 | N% 6,18 | S% 7,08 | Cl% 7,82 |
| Trouvé : | 69,2 | 6,5 | 6,2 | 7,0 | 8,1 |

**Exemple 3 : Trans (±) N-[2,3-dihydro 2-(1-pyrrolidinyl) 1H-inden-1-yl] N-méthyl 1-naphtalène acétamide et son chlorhydrate.**

On opère comme au stade C de l'exemple 1 à partir de 0,432 g de produit préparé comme au stade B, 0,484 g d'acide 1-naphtylacétique et 0,422 g de carbonyldiimidazole. On obtient 0,718 g de base ( F ≃ 127° C) puis 0,533 g de chlorhydrate attendu à partir de 0,655 g de base. F ≃ 240° C.

Analyse : $C_{26}H_{28}N_2O$, HCl : 420,986

| | | | | |
|---|---|---|---|---|
| Calculé : | C% 74,18 | H% 6,94 | N% 6,65 | Cl% 8,42 |
| Trouvé : | 74,2 | 7,1 | 6,8 | 8,3 |

**Exemple 4 : Trans (±) N-[2,3-dihydro 2-(1-pyrrolidinyl) 1H-inden-1-yl] N-méthyl 2-naphtalène acétamide et son chlorhydrate.**

En utilisant les mêmes quantités opératoires qu'à l'exemple 3 mais à partir d'acide 2-naphtyl acétique, on obtient la base puis 0,805 g de chlorhydrate attendu. F = 255° C après recristallisation dans l'isopropanol.

Analyse : $C_{26}H_{28}N_2O$, HCl : 420,986

| | | | | |
|---|---|---|---|---|
| Calculé : | C% 74,18 | H% 6,94 | N% 6,65 | Cl% 8,42 |
| Trouvé : | 74,4 | 7,0 | 6,5 | 8,6 |

On a préparé aussi de manière analogue à celle décrite dans les exemples précédents, le produit suivant :
- le maléate du trans (±) N-[2,3-dihydro 2-(1-pyrrolidinyl) 1H-inden-1-yl] N-méthyl 1H-indol 4-acétamide. F = 202° C-204° C.

**Exemple 5 :**

On a préparé des comprimés répondant à la formule suivante :

- produit de l'exemple 1 .................................... 200 mg
- excipient q.s.p.................................................. 800 mg

(détail de l'excipient : lactose, talc, amidon, stéarate de magnésium).

**Exemple 6 :**

On a préparé un soluté injectable (voie intra-musculaire) : répondant à la formule suivante :

- produit de l'exemple 1 .................................... 50 mg
- solvant stérile............................................ 5 cm3.

**ETUDE PHARMACOLOGIQUE**

1) Liaison au récepteur opiacé K in vitro .

On utilise des culots membranaires conservés à -30° C pendant environ 30 jours et préparés à partir de cervelets de cobayes.

Ces culots sont remis en suspension dans le tampon Tris pH 7,7. On répartit des fractions de 2 ml dans des tubes à hémolyse et ajoute de la $9^3H$ ethylkétocyclazocine 1nM et le produit à étudier. (Le produit est d'abord testé à $5\times10^{-6}$M (en triple). Lorsque le produit testé déplace de plus de 50 % la radioactivité liée spécifiquement au récepteur, il est testé à nouveau selon une gamme de 7 doses afin de déterminer la dose qui inhibe de 50 % la radioactivité liée spécifiquement au récepteur. On détermine ainsi la concentration inhibitrice 50 %).

La liaison non spécifique est déterminée par addition de produit connu sous le nom U-50488 H) (Lahti et al. 1982, Life Sci. 31, 2257) à $10^{-5}$M (en triple). On incube à 25° C pendant 40 minutes, remet au bain-marie à 0° C, 5 minutes, filtre sous vide, rince au tampon Tris pH 7,7 et compte la radioactivité en présence de scintillant Trition.

Le résultat est exprimé directement en concentration inhibitrice 50 % ($CI_{50}$, (c'est-à-dire en concentration de produit étudié, exprimée en nM, nécessaire pour déplacer 50 % de la radioactivité spécifique fixée sur le récepteur étudié).

Résultat :

La $CI_{50}$ trouvée pour le produit de l'exemple 1 est de 1,8 nanomoles et de 4,9 nanomoles pour le produit de l'exemple 3.

2) Activité analgésique in vivo
- test de la plaque chaude .

Des souris femelles pesant 22 à 24 g sont placées une par une sur une plaque de cuivre maintenue à 56° C : la réaction à la douleur se manifeste par le léchage des pattes avant de l'animal ; le temps de cette réaction est noté et l'on ne retient que les souris réagissant en moins de 8 secondes.

Les animaux sont répartis par groupes homogènes et traités par le produit à étudier administré par voie sous cutanée, un groupe ne recevant que le véhicule. Le temps de réaction à la douleur est de nouveau mesuré 30 à 60 minutes après le traitement. La dose active ou $DA_{100}$ est la dose qui augmente le temps de réaction de 100%, 30 minutes après le traitement compte tenu des variations du temps de réaction des animaux témoins.

Pour le produit de l'exemple 1, la $DA_{100}$ est de 8 mg/kg.

3) Action antiarythmique chez le rat .

On trachéotomise des rats mâles pesant 300-350 g anesthésiés par voie intrapéritonéale à l'aide de 1,20 g/kg d'uréthane et les soumet à une respiration artificielle (40-50 insufflations de 3 ml/minute).

On implante des aiguilles en sous cutané de manière à enregistrer l'électrocardiogramme des rats sur

le signal en dérivation DII.

On administre les produits à tester par voie intraveineuse.

Cinq minutes après l'administration du produit, on perfuse la veine jugulaire des rats avec 10 ug/mn sous 0,2 ml d'une solution d'aconitine et on note le temps d'apparition des troubles du rythme cardiaque.

Les résultats sont exprimés en pourcentage d'allongement du temps d'apparition des troubles du rythme cardiaque par rapport aux témoins et en fonction de la dose du produit testé.

Les résultats figurant sur le tableau ci-après montrent que certains des produits de la présente demande sont doués de bonnes propriétés antiarythmiques.

| Produit de l'exemple | Dose mg/kg | Pourcentage d'allongement du temps |
|---|---|---|
| 1 | 10 | +65 % |

4) Mesure de l'activité diurétique .

Des rats mâles, de souche Sprague Dawley et de poids 180-200 g, sont mis à jeun 17 heures avant l'essai, tout en recevant de l'eau ad libitum.

Des lots de 8 animaux sont constitués par dose testée. Les rats reçoivent le produit à tester ou son véhicule par voie orale.

Le volume urinaire est mesuré toutes les heures pendant les 2 heures qui suivent l'administration du produit. A la fin de cette période, les urines sont recueillies et l'activité du produit est exprimée en pourcentage de variation calculé sur le volume urinaire correspondant à la périod $t_{1h}$-$t_{2h}$.

| Produit de l'exemple | Dose mg/kg | Pourcentage d'allongement du temps |
|---|---|---|
| 1 | 1 | + 131 |
| 3 | | + 267 |

5) Etude de la toxicité aigüe .

On a évalué les doses létales $DL_0$ des différents composés testés après administration per os chez la souris.

On appelle $DL_0$ la dose maximale ne provoquant aucune mortalité en 8 jours.

Résultats :

| Produit de l'exemple | $DL_0$ |
|---|---|
| 1 | 100 mg/kg |
| 3 | 100 mg/kg |
| 4 | 200 mg/kg |

## Revendications

1. Composés de formule (I) :

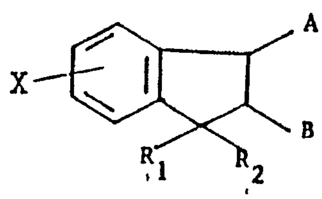

(I)

dans laquelle X représente un atome d'hydrogène, un atome d'halogène, un radical alkyle renfermant de 1 à 5 atomes de carbone, un radical alkoxy renfermant de 1 à 5 atomes de carbone, $R_1$ et $R_2$, identiques ou différents, représentent un atome d'hydrogène ou un radical alkyle renfermant de 1 à 5 atomes de carbone et A et B sont tels que l'un des substituants A ou B est choisi dans :
- le groupe de formule :

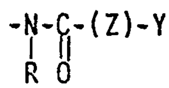

R étant un atome d'hydrogène ou un radical alkyle renfermant de 1 à 5 atomes de carbone, Z une chaîne alkylène linéaire $-(CH_2)_n-$, n étant un nombre entier pouvant varier entre 0 et 5 ou une chaîne alkylène ramifiée renfermant de 2 à 8 atomes de carbone, Y représentant un radical naphtyle ou indényle ou un radical hétéromonocyclique renfermant 5 ou 6 chaînons ou hétérobicyclique, tous les radicaux mentionnés précédemment étant substitués éventuellement par un ou plusieurs radicaux choisis parmi les radicaux alkyle ou alkoxy renfermant de 1 à 5 atomes de carbone, halogène, hydroxyle, trifluorométhyle, nitro, amino, monoalkyl- ou dialkylamino ou phényle lui-même substitué éventuellement par un ou plusieurs radicaux choisis parmi les radicaux alkyle ou alkoxy renfermant de 1 à 4 atomes de carbone et halogène et l'autre des substituants A ou B est choisi dans
- le groupe de formule :

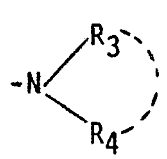

$R_3$ et $R_4$, identiques ou différents, représentant un atome d'hydrogène ou un radical alkyle renfermant de 1 à 5 atomes de carbone ou $R_3$ et $R_4$ forment ensemble avec l'atome d'azote auquel ils sont liés un hétérocycle à 5 ou 6 chaînons comportant éventuellement un autre hétéroatome tel que l'oxygène ou l'azote ou le soufre, lesdits composés de formule (I) pouvant être dans toutes les formes énantiomères

et diastéréoisomères possibles et sous forme de sels d'addition avec les acides.

2. Composés de formule (I), tels que définis à la revendication 1, caractérisés en ce que les groupements A et B sont en configuration trans ainsi que leurs sels d'addition avec les acides.

3. Composés de formule (I), tels que définis à la revendication 1 ou 2, caractérisés en ce que dans le groupe

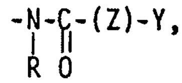

R représente un atome d'hydrogène, un radical méthyle ou éthyle, Z représente un groupe

$-CH_2-$ ou 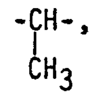

Y représente un radical naphtyle, indényle, benzo [b] thiényle, indolyle, benzofurannyle, quinolyle, benzimidazolyle, benzoxazolyle, benzothiazolyle, thiényle éventuellement substitué par un radical phényle, ainsi que leurs sels d'addition avec les acides.

4. Composés de formule (I), tels que définis à l'une quelconque des revendications 1 à 3, caractérisés en ce que X, $R_1$ et $R_2$ représentent un atome d'hydrogène et en ce que dans le groupe :

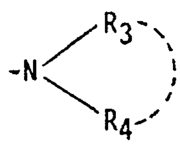 ,

$R_3$ et $R_4$ forment avec l'atome d'azote auquel ils sont liés l'hétérocycle pyrrolidine, ainsi que leurs sels d'addition avec les acides.

5. le (trans) (±) N-[2,3-dihydro 2-(1-pyrrolidinyl) 1H-inden-1-yl] N-méthyl 4-benzo [b] thiophène acétamide et ses sels avec les acides,
et le trans (±) n-[2,3-dihydro 2-(1-pyrrolidinyl) 1H-inden-1-yl] N-méthyl 1-naphtalène acétamide et ses sels d'addition avec les acides.

6. Procédé de préparation des produits de formule (I) dont les groupements A et B sont en configuration trans, caractérisé en ce que l'on condense le produit de formule (II) :

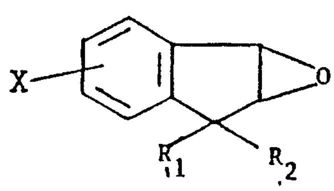

(II)

X, $R_1$ et $R_2$ ayant les significations données précédemment,
- soit avec une amine de formule (III) :

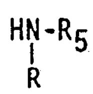

(III)

R ayant la signification donnée précédemment et $R_5$ étant un groupement protecteur de la fonction amine et notamment un groupement benzyle, pour obtenir un produit de formule (IV) :

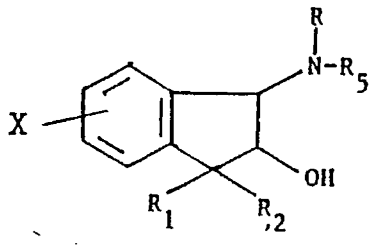

(IV)

dont on active la fonction hydroxyle et que l'on condense avec une amine de formule (V) :

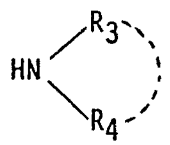

(V)

dans laquelle $R_3$ et $R_4$ ont les significations indiquées précédemment pour obtenir un produit de formule (VI) :

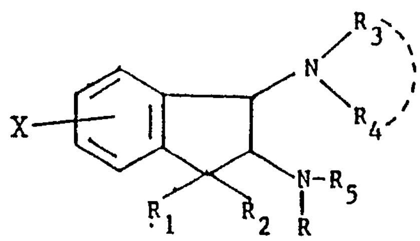

(VI)

dont on élimine le groupement protecteur $R_5$ de la fonction amine pour obtenir un produit de formule (VII) :

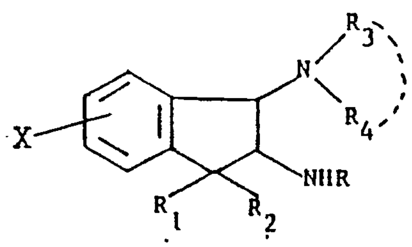

(VII)

que l'on traite avec un acide de formule (VIII) ou un dérivé fonctionnel de cet acide :

$$Y-(Z)-COOH \qquad (VIII)$$

dans laquelle Y et Z, ont les significations précédentes pour obtenir un produit de formule (I) dans laquelle A représente :

le groupe 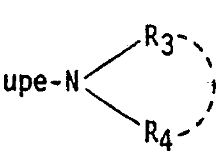 et B le groupe 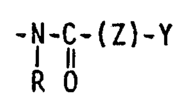

- soit avec une amine de formule (V) :

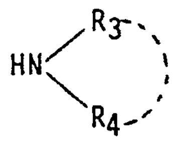

(V)

dans laquelle $R_3$ et $R_4$ ont la signification précédente pour obtenir un produit de formule (IX) :

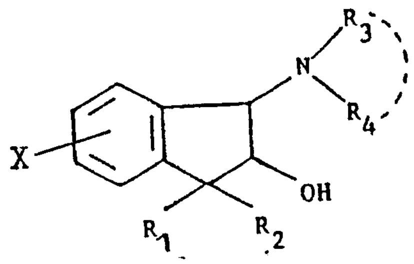

(IX)

dont on active la fonction hydroxyle et que l'on traite avec une amine de formule (X) :

$NH_2R$ (X)

dans laquelle R a la signification précédente pour obtenir un produit de formule (XI) :

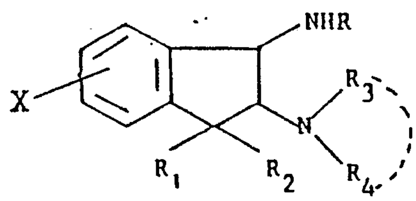

(XI)

que l'on condense avec un acide de formule (VIII) ou un dérivé fonctionnel de cet acide :

Y-(Z)-COOH (VIII)

dans laquelle Z et Y ont les significations précédentes pour obtenir un produit de formule (I) dans laquelle A représente :

le groupe 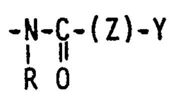 et B le groupe 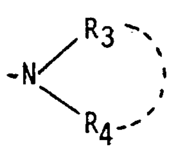 ,

lesdits composés de formule (I) pouvant être dédoublés pour obtenir les formes optiquement actives et que l'on traite, si désiré, avec un acide minéral ou organique, pour obtenir un sel.

7. A titre de médicaments, les produits de formule (I), tels que définis à l'une quelconque des revendications 1 à 4, ainsi que leurs sels d'addition avec les acides pharmaceutiquement acceptables.

8. A titre de médicament, les produits de la revendication 5, ainsi que leurs sels d'addition avec les acides pharmaceutiquement acceptables.

9. Les compositions pharmaceutiques contenant à titre de principe actif, l'un au moins des médicaments tels que définis par la revendication 7 ou 8.

**10.** A titre de produits intermédiaires, les produits de formules (IV), (VI), (VII), (IX), (XI) dans lesquelles X ne représente pas un atome d'hydrogène.

Revendications pour l'Etat contractant suivant : ES

**1.** Procédé de préparation des dérivés de l'indane et leurs sels d'addition avec les acides minéraux ou organiques répondant à la formule (I):

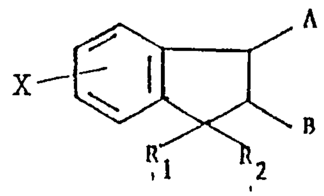

(I)

dans laquelle X représente un atome d'hydrogène, un atome d'halogène, un radical alkyle renfermant de 1 à 5 atomes de carbone, un radical alkoxy renfermant de 1 à 5 atomes de carbone, $R_1$ et $R_2$, identiques ou différents, représentent un atome d'hydrogène ou un radical alkyl renfermant de 1 à 5 atomes de carbone et A et B sont tels que l'un des substituants A ou B est choisi dans :
- le groupe de formule :

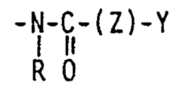

R étant un atome d'hydrogène ou un radical alkyle renfermant de 1 à 5 atomes de carbone, Z une chaîne alkylène linéaire $-(CH_2)_n-$, n étant un nombre entier pouvant varier entre 0 et 5 ou une chaîne alkylène ramifiée renfermant de 2 à 8 atomes de carbone, Y représentant un radical naphtyle ou indényle ou un radical hétéromonocyclique renfermant 5 ou 6 chaînons ou hétérobicyclique, tous les radicaux mentionnés précédemment étant substitués éventuellement par un ou plusieurs radicaux choisis parmi les radicaux alkyle ou alkoxy renfermant de 1 à 5 atomes de carbone, halogène, hydroxyle, trifluorométhyle, nitro, amino, monoalkyl- ou dialkylamino ou phényle lui-même substitué éventuellement par un ou plusieurs radicaux choisis parmi les radicaux alkyle ou alkoxy renfermant de 1 à 4 atomes de carbone et halogène et l'autre des substituants A ou B est choisi dans
- le groupe de formule :

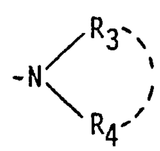

$R_3$ et $R_4$ identiques ou différents, représentant un atome d'hydrogène ou un radical alkyle renfermant de 1 à 5 atomes de carbone ou $R_3$ et $R_4$ forment ensemble avec l'atome d'azote auquel ils sont liés un hétérocycle à 5 ou 6 chaînons comportant éventuellement un autre hétéroatome tel que l'oxygène ou l'azote ou le soufre, lesdits composés de formule (I) pouvant être dans toutes les formes énantiomères et diastéréoisomères possibles et sous forme de sels d'addition avec les acides, caractérisé en ce que l'on condense le produit de formule (II) :

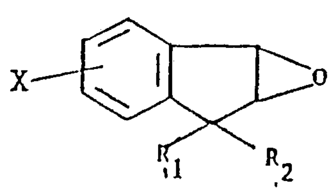

(II)

X, $R_1$ et $R_2$ ayant les significations données précédemment,

- soit avec une amine de formule (III) :

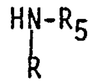

(III)

R ayant la signification donnée précédemment et $R_5$ étant un groupement protecteur de la fonction amine et notamment un groupement benzyle, pour obtenir un produit de formule (IV) :

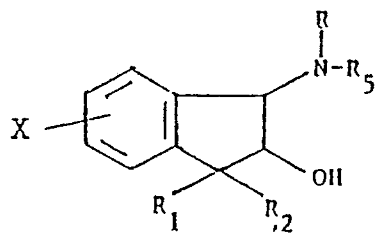

(IV)

dont on active la fonction hydroxyle et que l'on condense avec une amine de formule (V) :

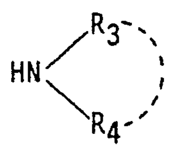

(V)

dans laquelle $R_3$ et $R_4$ ont les significations indiquées précédemment pour obtenir un produit de formule (VI) :

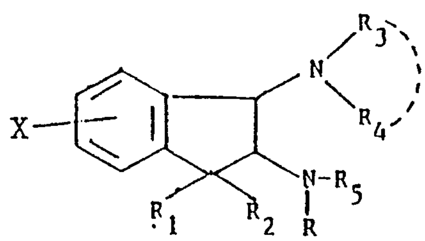

(VI)

dont on élimine le groupement protecteur $R_5$ de la fonction amine pour obtenir un produit de formule (VII) :

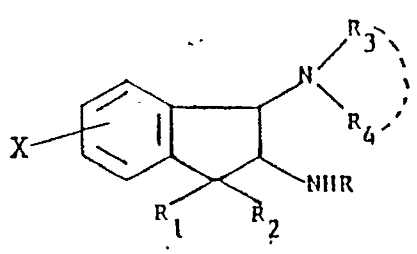

(VII)

que l'on traite avec un acide de formule (VIII) ou un dérivé fonctionnel de cet acide :

$$Y\text{-}(Z)\text{-}COOH \qquad (VIII)$$

dans laquelle Y et Z, ont les significations précédentes pour obtenir un produit de formule (I) dans laquelle A représente :

le groupe 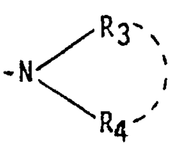 et B le groupe 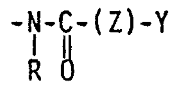

- soit avec une amine de formule (V) :

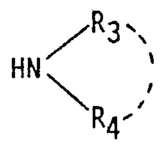 (V)

dans laquelle $R_3$ et $R_4$ ont la signification précédente pour obtenir un produit de formule (IX) :

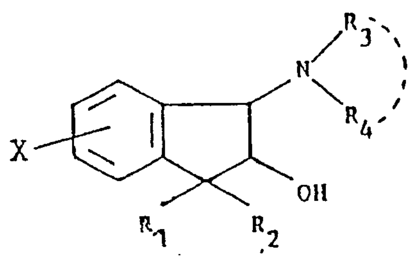 (IX)

dont on active la fonction hydroxyle et que l'on traite avec une amine de formule (X) :

$NH_2R$ (X)

dans laquelle R a la signification précédente pour obtenir un produit de formule (XI) :

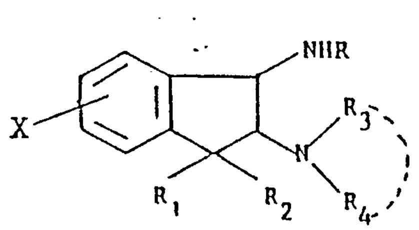 (XI)

que l'on condense avec un acide de formule (VIII) ou un dérivé fonctionnel de cet acide :

Y-(Z)-COOH (VIII)

dans laquelle Z et Y ont les significations précédentes pour obtenir un produit de formule (I) dans laquelle A représente :

le groupe 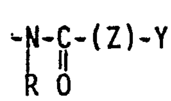 et B le groupe 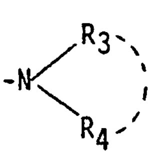 ,

lesdits composés de formule (I) pouvant être dédoublés pour obtenir les formes optiquement actives et que l'on traite, si désiré, avec un acide minéral ou organique, pour obtenir un sel.

2. Procédé selon la revendication 1, caractérisé en ce que l'on utilise une amine de formule (III) ou (X)

dans laquelle R représente un atome d'hydrogène, un radical méthyle ou éthyle et un acide de formule (VIII) dans laquelle Z représente un groupe

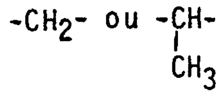

et Y représente un radical naphtyle, indényle, benzo [b], thiényle, indolyle, benzofurannyl e, quinolyle, benzimidazolyle, benzoxazolyle, benzothiazolyle, thiényle éventuellement substitué par un radical phényle.

3. Procédé selon la revendication 1 ou 2, caractérisé en ce que l'on utilise au départ un produit de formule (II), dans laquelle X, $R_1$ et $R_2$ représentent un atome d'hydrogène et une amine de formule (V) dans laquelle $R_3$ et $R_4$ forment avec l'atome d'azote auquel ils sont liés l'hétérocycle pyrrolidine.

Revendications pour l'Etat contractant suivant : GR

1. Procédé de préparation des dérivés de l'indane et leurs sels d'addition avec les acides minéraux ou organiques répondant à la formule (I):

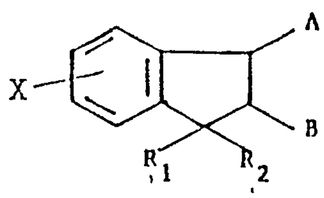

(I)

dans laquelle X représente un atome d'hydrogène, un atome d'halogène, un radical alkyle renfermant de 1 à 5 atomes de carbone, un radical alkoxy renfermant de 1 à 5 atomes de carbone, $R_1$ et $R_2$, identiques ou différents, représentent un atome d'hydrogène ou un radical alkyle renfermant de 1 à 5 atomes de carbone et A et B sont tels que l'un des substituants A ou B est choisi dans :
- le groupe de formule :

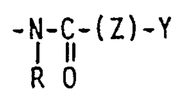

R étant un atome d'hydrogène ou un radical alkyle renfermant de 1 à 5 atomes de carbone, Z une chaîne alkylène linéaire $-(CH_2)_n-$, n étant un nombre entier pouvant varier entre 0 et 5 ou une chaîne alkylène ramifiée renfermant de 2 à 8 atomes de carbone, Y représentant un radical naphtyle ou indényle ou un radical hétéromonocyclique renfermant 5 ou 6 chaînons ou hétérobicyclique, tous les radicaux mentionnés précédemment étant substitués éventuellement par un ou plusieurs radicaux choisis parmi les radicaux alkyle ou alkoxy renfermant de 1 à 5 atomes de carbone, halogène, hydroxyle, trifluorométhyle, nitro, amino, monoalkyl- ou dialkylamino ou phényle lui-même substitué éventuellement par un ou plusieurs radicaux choisis parmi les radicaux alkyle ou alkoxy renfermant de 1 à 4 atomes de carbone et halogène et l'autre des substituants A ou B est choisi dans
- le groupe de formule :

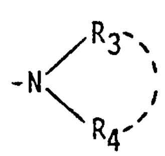

$R_3$ et $R_4$ identiques ou différents, représentant un atome d'hydrogène ou un radical alkyle renfermant de 1 à 5 atomes de carbone ou $R_3$ et $R_4$ forment ensemble avec l'atome d'azote auquel ils sont liés un hétérocycle à 5 ou 6 chaînons comportant éventuellement un autre hétéroatome tel que l'oxygène ou

l'azote ou le soufre, lesdits composés de formule (I) pouvant être dans toutes les formes énantiomères et diastéréoisomères possibles et sous forme de sels d'addition avec les acides, caractérisé en ce que l'on condense le produit de formule (II) :

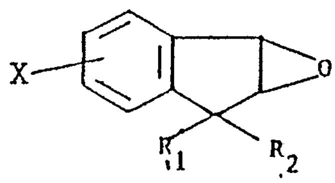

(II)

X, $R_1$ et $R_2$ ayant les significants données précédemment,
- soit avec une amine de formule (III) :

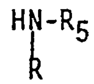

(III)

R ayant la signification donnée précédemment et $R_5$ étant un groupement protecteur de la fonction amine et notamment un groupement benzyle, pour obtenir un produit de formule (IV) :

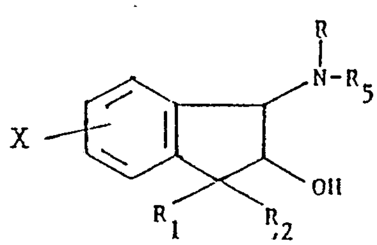

(IV)

dont on active la fonction hydroxyle et que l'on condense avec une amine de formule (V) :

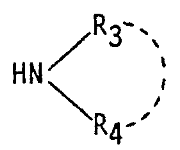

(V)

dans laquelle $R_3$ et $R_4$ ont les significations indiquées précédemment pour obtenir un produit de formule (VI) :

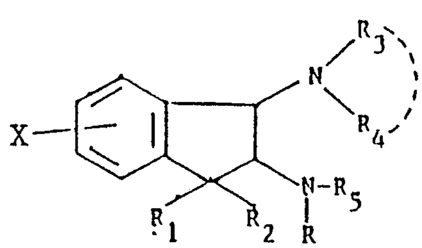

(VI)

dont on élimine le groupement protecteur $R_5$ de la fonction amine pour obtenir un produit de formule (VII) :

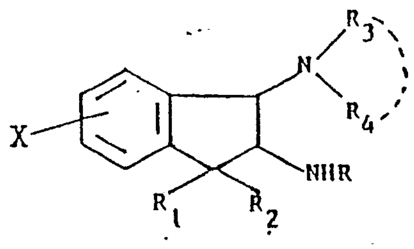

(VII)

que l'on traite avec un acide de formule (VIII) on un dérivé fonctionnel de cet acide :

Y-(Z)-COOH (VIII)

dans laquelle Y et Z, ont les significations précédentes pour obtenir un produit de formule (I) dans laquelle A représente :

le groupe 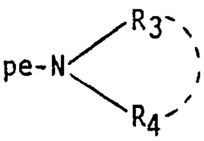 et B le groupe 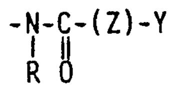

- soit avec une amine de formule (V) :

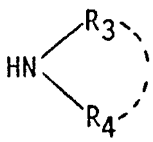

(V)

dans laquelle $R_3$ et $R_4$ ont la signification précédente pour obtenir un produit de formule (IX) :

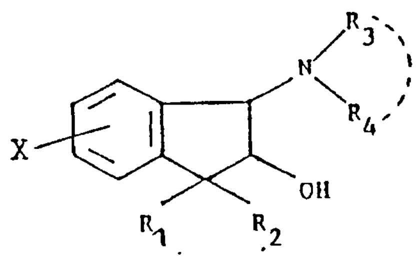

(IX)

dont on active la fonction hydroxyle et que l'on traite avec une amine de formule (X) :

$NH_2R$ (X)

dans laquelle R a la signification précédente pour obtenir un produit de formule (XI) :

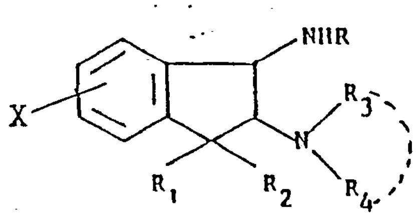

(XI)

que l'on condense avec un acide de formule (VIII) ou un dérivé fonctionnel de cet acide :

Y-(Z)-COOH (VIII)

dans laquelle Z et Y ont les significations précédentes pour obtenir un produit de formule (I) dans laquelle A représente :

le groupe 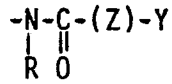 et B le groupe 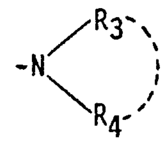 ,

lesdits composés de formule (I) pouvant être dédoublés pour obtenir les formes optiquement actives et que l'on traite, si désiré, avec un acide minéral ou organique, pour obtenir un sel.

2. Procédé selon la revendication 1, caractérisé en ce que l'on utilise une amine de formule (III) ou (X) dans laquelle R représente un atome d'hydrogène, un radical méthyle ou éthyle et un acide de formule (VIII) dans laquelle Z représente un groupe

$-CH_2-$ ou 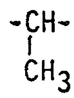

et Y représente un radical naphtyle, indényle, benzo [b] thiényle, indolyle, benzofurannyle, quinolyle, benzimidazolyle, benzoxazolyle, benzothiazolyle, thiényle éventuellement substitué par un radical phényle.

3. Procédé selon la revendication 1 ou 2, caractérisé en ce que l'on utilise au départ un produit de formule (II), dans laquelle X, $R_1$ et $R_2$ représentent un atome d'hydrogène et une amine de formule (V) dans laquelle $R_3$ et $R_4$ forment avec l'atome d'azote auquel ils sont liés l'hétérocycle pyrrolidine.

4. Procédé selon l'une quelconque des revendications 1 à 3, caractérisé en ce que l'on prépare le (trans) (±) N-[2,3-dihydro 2-(1-pyrrolidinyl 1H-inden 1-yl] N-méthyl 4-benzo [b] thiophène acétamide et ses sels avec les acides,
et le trans (±) N-[2,3-dihydro 2-(1-pyrrolidinyl) 1H-inden-1-yl] N-méthyl 1-naphtalène acétamide et ses sels d'addition avec les acides.

5. Procédé de préparation de compositions pharmaceutiques caractérisé en ce que l'on met à titre de principe actif, l'un au moins des produits de formule (I) telle que définie à la revendication 1 ou l'un au moins de leurs sels d'addition avec les acides pharmaceutiquement acceptables sous une forme destinée à cet usage.

6. A titre de composés industriels nouveaux, les produits de formule (IV), (VI), (VII), (IX), (XI) tels que définis à la revendication 1, dans lesquels X ne représente pas un atome d'hydrogène.

## Claims

1. compounds of formula (I):

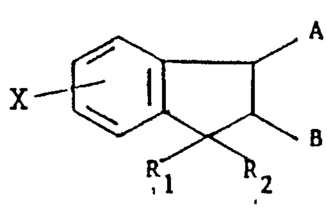

(I)

in which X represents a hydrogen atom, a halogen atom, an alkyl radical containing 1 to 5 carbon

atoms, an alkoxy radical containing 1 to 5 carbon atoms, $R_1$ and $R_2$, identical or different, represent a hydrogen atom or an alkyl radical containing 1 to 5 carbon atoms and A and B are such that one of the substituents A or B is chosen from:
- the group of formula:

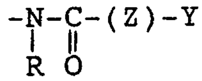

R being a hydrogen atom or an alkyl radical containing 1 to 5 carbon atoms, Z a linear alkylene chain -- $(CH_2)n$, n being an integer which can vary between 0 and 5 or a branched alkylene chain containing 2 to 8 carbon atoms, Y representing a naphthyl or indenyl radical or a heteromonocyclic radical containing 5 or 6 links or a heterobicyclic radical, all the radicals mentioned previously being optionally substituted by one or more radicals chosen from the following radicals: alkyl or alkoxy containing 1 to 5 carbon atoms, halogen, hydroxyl, trifluoromethyl, nitro, amino, monoalkyl- or dialkylamino or phenyl, itself optionally substituted by one or more radicals chosen from alkyl or alkoxy radicals containing 1 to 4 carbon atoms and halogen atoms, and the other substituent A or B is chosen from
- the group of formula:

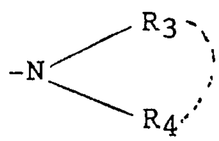

$R_3$ and $R_4$, identical or different, representing a hydrogen atom or an alkyl radical containing 1 to 5 carbon atoms or $R_3$ and $R_4$ form, together with the nitrogen atom to which they are linked, a heterocycle with 5 or 6 links optionally comprising another heteroatom such as oxygen or nitrogen or sulphur, the said compounds of formula (I) being able to be in all the possible enantiomer and diastereoisomer forms and in the form of addition salts with acids.

**2.** Compounds of formula (I) as defined in claim 1, characterized in that the groups A and B are of trans configuration, as well as their addition salts with acids.

**3.** Compounds of formula (I), as defined in claim 1 or 2, characterized in that in the group

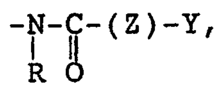

R represents a hydrogen atom, a methyl or ethyl radical, Z represents a

$-CH_2-$ or 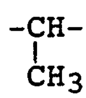

group, Y represents one of the following radicals: naphthyl, indenyl, benzo-[b]-thienyl, indolyl, benzofurannyl, quinolyl, benzimidazolyl, benzoxazolyl, benzothiazolyl, thienyl optionally substituted by a phenyl radical, as well as their addition salts with acids.

**4.** Compounds of formula (I), as defined in any one of claims 1 to 3, characterized in that X, $R_1$ and $R_2$ represent a hydrogen atom and in that in the group:

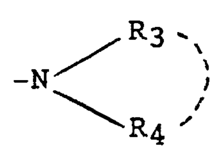

$R_3$ and $R_4$ form, together with the nitrogen atom to which they are linked, the pyrrolidine heterocycle, as well as their addition salts with acids.

5. (trans) (±) N-[2,3-dihydro-2-(1-pyrrolidinyl)-1H-inden-1-yl]-N-methyl-4-benzo-[b]-thiophene acetamide and its salts with acids,
and trans (±) N-[2,3-dihydro-2-(1-pyrrolidinyl)-1H-inden-1-yl]-N-methyl-1-naphthalene acetamide and its addition salts with acids.

6. Preparation process for the products of formula (I) of which the A and B groups are of trans configuration, characterized in that the product of formula (II):

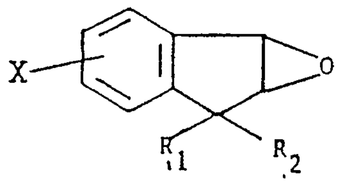

(II)

X, $R_1$ and $R_2$ having the meanings given previously, is condensed:
- either with an amine of formula (III):

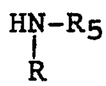

(III)

R having the previously given meaning and $R_5$ being a protector group of the amine function and notably a benzyl group, in order to obtain a product of formula (IV):

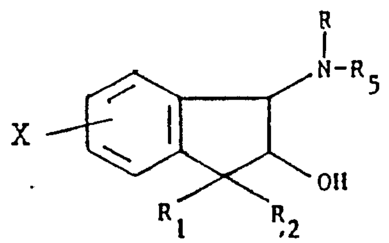

(IV)

of which the hydroxyl function is activated and which is condensed with an amine of formula (V):

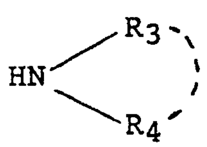

(V)

in which $R_3$ and $R_4$ have the previously indicated meanings, in order to obtain a product of formula (VI):

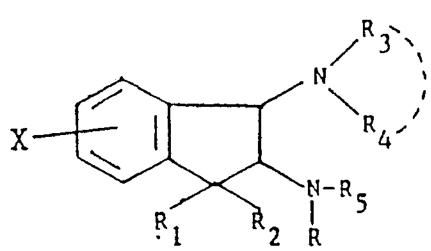

(VI)

of which the protector group $R_5$ of the amine function is eliminated to obtain a product of formula (VII):

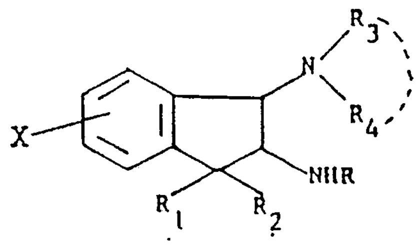

(VII)

which is treated with an acid of formula (VIII) or a functional derivative of this acid:

$$Y\text{-}(Z)\text{-}COOH \qquad (VIII)$$

in which Y and Z have the previous meanings, to obtain a product of formula (I) in which A represents:

the group 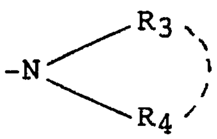 and B the group 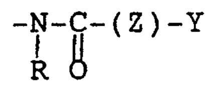

- or with an amine of formula (V):

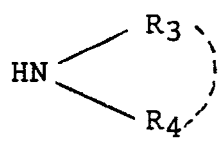

(V)

in which $R_3$ and $R_4$ have the previous meaning, to obtain a product of formula (IX):

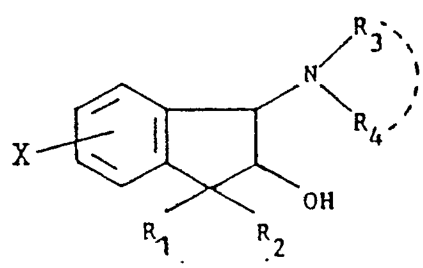

(IX)

of which the hydroxyl function is activated and which is treated with an amine of formula (X):

$$NH_2R \qquad (X)$$

in which R has the previous meaning, to obtain a product of formula (XI):

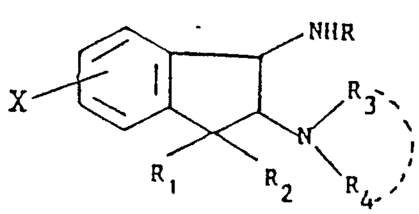

(XI)

which is condensed with an acid of formula (VIII) or a functional derivative of this acid:

Y-(Z)-COOH (VIII)

in which Z and Y have the previous meanings, to obtain a product of formula (I) in which A represents:

the group 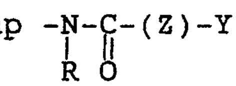 and B the group 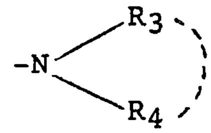

the said compounds of formula (I) being able to be resolved in order to obtain the optically active forms, and which are treated, if desired, with a mineral or organic acid, in order to obtain the salt.

7. As medicaments, the products of formula (I), as defined in any one of claims 1 to 4, as well as their addition salts with pharmaceutically acceptable acids.

8. As medicaments, the products of claim 5, as well as their addition salts with pharmaceutically acceptable acids.

9. The pharmaceutical compositions containing as active ingredient at least one of the medicaments as defined by claim 7 or 8.

10. As intermediate products, the products of formula (IV), (VI), (VII), (IX), (XI) in which X does not represent a hydrogen atom.

Claims for the following Contracting State : ES

1. Preparation process for derivatives of indane and their addition salts with mineral or organic acids corresponding to the formula (I):

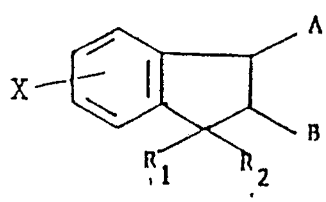

(I)

in which X represents a hydrogen atom, a halogen atom, an alkyl radical containing 1 to 5 carbon atoms, an alkoxy radical containing 1 to 5 carbon atoms, $R_1$ and $R_2$, identical or different, represent a hydrogen atom or an alkyl radical containing 1 to 5 carbon atoms and A and B are such that one of the substituents A or B is chosen from:
- the group of formula:

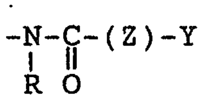

R being a hydrogen atom or an alkyl radical containing 1 to 5 carbon atoms, Z a linear alkylene chain $-(CH_2)_n$, n being an integer which can vary between 0 and 5 or a branched alkylene chain containing 2 to 8 carbon atoms, Y representing a naphthyl or indenyl radical or a heteromonocyclic radical containing 5 or 6 links or a heterobicyclic radical, all the radicals mentioned previously being optionally substituted by one or more radicals chosen from the following radicals: alkyl or alkoxy containing 1 to 5 carbon atoms, halogen, hydroxyl, trifluoromethyl, nitro, amino, monoalkyl- or dialkylamino or phenyl, itself optionally substituted by one or more radicals chosen from alkyl or alkoxy radicals containing 1 to 4 carbon atoms and halogen atoms, and the other substituent A or B is chosen from

- the group of formula:

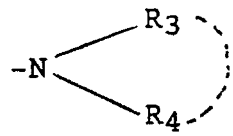

$R_3$ and $R_4$, identical or different, representing a hydrogen atom or an alkyl radical containing 1 to 5 carbon atoms or $R_3$ and $R_4$ form, together with the nitrogen atom to which they are linked, a heterocycle with 5 or 6 links optionally comprising another heteroatom such as oxygen or nitrogen or sulphur, the said compounds of formula (I) being able to be in all the possible enantiomer and diastereoisomer forms and in the form of addition salts with acids, characterized in that the product of formula (II):

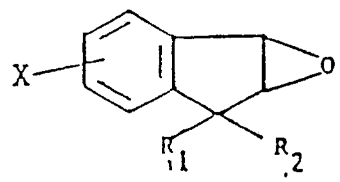

(II)

X, $R_1$ and $R_2$ having the previously given meanings, is condensed:
- <u>either</u> with an amine of formula (III):

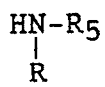

(III)

R having the previously given meaning and $R_5$ being a protector group of the amine function and notably a benzyl group, in order to obtain a product of formula (IV):

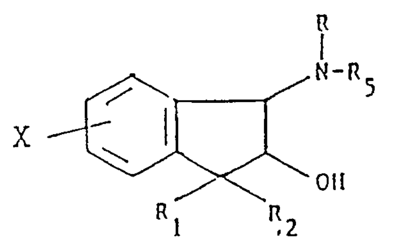

(IV)

of which the hydroxyl function is activated and which is condensed with an amine of formula (V):

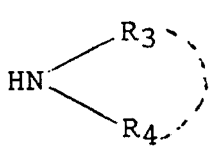

(V)

in which $R_3$ and $R_4$ have the previously indicated meanings, in order to obtain a product of formula (VI):

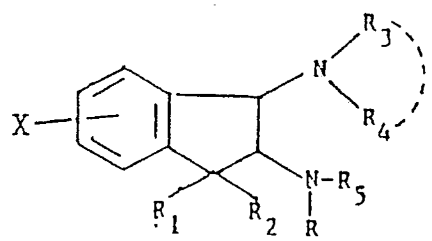

(VI)

of which the protector group $R_5$ of the amine function is eliminated to obtain a product of formula (VII):

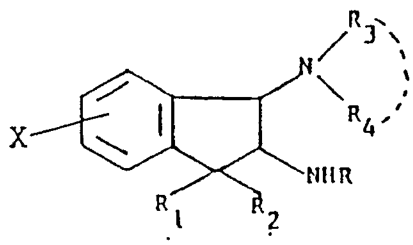

(VII)

which is treated with an acid of formula (VIII) or a functional derivative of this acid:

$$Y-(Z)-COOH \qquad (VIII)$$

in which Y and Z have the previous meanings, to obtain a product of formula (I) in which A represents:

the group 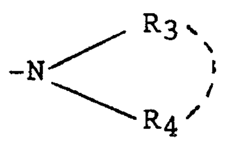 and B the group 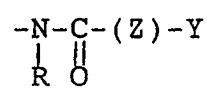

- or with an amine of formula (V):

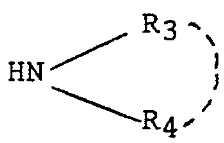

(V)

in which $R_3$ and $R_4$ have the previous meaning, to obtain a product of formula (IX):

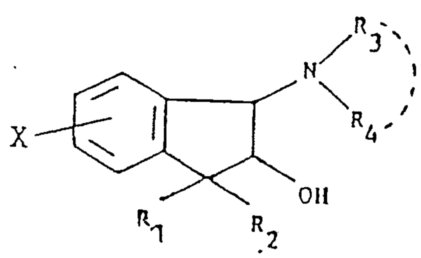

(IX)

of which the hydroxyl function is activated and which is treated with an amine of formula (X):

$$NH_2R \qquad (X)$$

in which R has the previous meaning, to obtain a product of formula (XI):

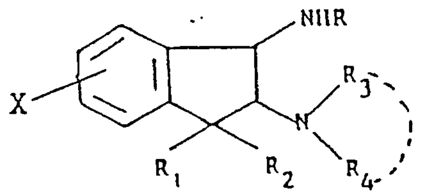

(XI)

which is condensed with an acid of formula (VIII) or a functional derivative of this acid:

$$Y\text{-}(Z)\text{-}COOH \qquad (VIII)$$

in which Z and Y have the previous meanings, to obtain a product of formula (I) in which A represents:

the group 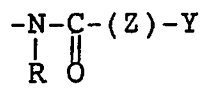 and B the group 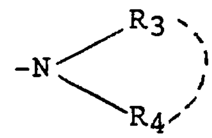

the said compounds of formula (I) being able to be resolved in order to obtain the optically active forms, and which are treated, if desired, with a mineral or organic acid, in order to obtain the salt.

2. Process according to claim 1, characterized in that an amine of formula (III) or (X) is used in which R represents a hydrogen atom, a methyl or ethyl radical, and an acid of formula (VIII) is used in which Z represents a $-CH_2-$ or

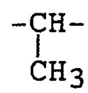

group and Y represents one of the following radicals: naphthyl, indenyl, benzo-[b]-thienyl, indolyl, benzofurannyl, quinolyl, benzimidazolyl, benzoxazolyl, benzothiazolyl, thienyl optionally substituted by a phenyl radical.

3. Process according to claim 1 or 2, characterized in that a product of formula (II), in which X, $R_1$ and $R_2$ represent a hydrogen atom, and an amine of formula (V) in which $R_3$ and $R_4$ form, together with the nitrogen atom to which they are linked, the pyrrolidine heterocycle, are used at the start.

Claims for the following Contracting State : GR

1. Preparation process for derivatives of indane and their addition salts with mineral or organic acids, corresponding to the formula (I):

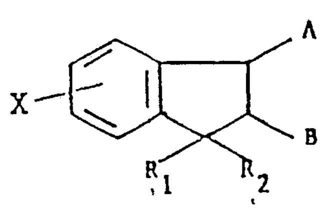

(I)

in which X represents a hydrogen atom, a halogen atom, an alkyl radical containing 1 to 5 carbon atoms, an alkoxy radical containing 1 to 5 carbon atoms, $R_1$ and $R_2$, identical or different, represent a hydrogen atom or an alkyl radical containing 1 to 5 carbon atoms and A and B are such that one of the substituents A or B is chosen from:
- the group of formula:

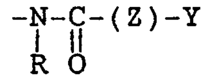

R being a hydrogen atom or an alkyl radical containing 1 to 5 carbon atoms, Z a linear alkylene chain --$(CH_2)n$, n being an integer which can vary between 0 and 5 or a branched alkylene chain containing 2 to 8 carbon atoms, Y representing a naphthyl or indenyl radical or a heteromonocyclic radical containing 5 or 6 links or a heterobicyclic radical, all the radicals mentioned previously being optionally substituted by one or more radicals chosen from the following radicals: alkyl or alkoxy containing 1 to 5 carbon atoms, halogen, hydroxyl, trifluoromethyl, nitro, amino, monoalkyl- or dialkylamino or phenyl, itself optionally substituted by one or more radicals chosen from alkyl or alkoxy radicals containing 1 to 4 carbon atoms and halogen atoms, and the other substituent A or B is chosen from
- the group of formula:

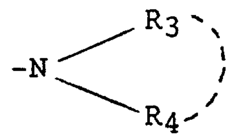

$R_3$ and $R_4$, identical or different, representing a hydrogen atom or an alkyl radical containing 1 to 5 carbon atoms or $R_3$ and $R_4$ form, together with the nitrogen atom to which they are linked, a heterocycle with 5 or 6 links optionally comprising another heteroatom such as oxygen or nitrogen or sulphur, the said compounds of formula (I) being able to be in all the possible enantiomer and diastereoisomer forms and in the form of addition salts with acids, characterized in that the product of formula (II):

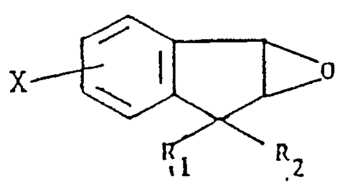

(II)

X, $R_1$ and $R_2$ having the previously given meanings, is condensed:
- <u>either</u> with an amine of formula (III):

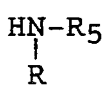

(III)

R having the previously given meaning and $R_5$ being a protector group of the amine function and notably a benzyl group, in order to obtain a product of formula (IV):

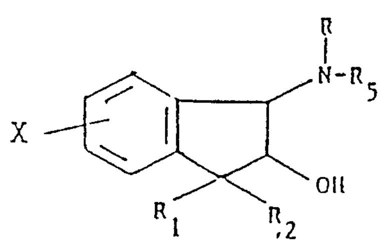

(IV)

of which the hydroxyl function is activated and which is condensed with an amine of formula (V):

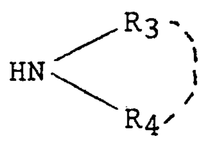

(V)

in which $R_3$ and $R_4$ have the previously indicated meanings, in order to obtain a product of formula (VI):

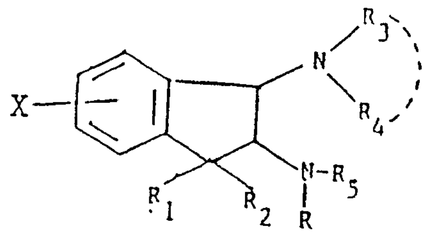

(VI)

of which the protector group $R_5$ of the amine function is eliminated to obtain a product of formula (VII):

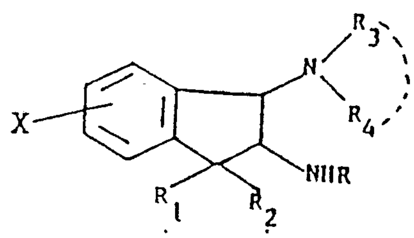

(VII)

which is treated with an acid of formula (VIII) or a functional derivative of this acid:

$$Y-(Z)-COOH \qquad (VIII)$$

in which Y and Z have the previous meanings, to obtain a product of formula (I) in which A represents:

the group 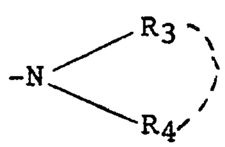 and B the group 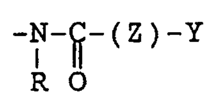

- <u>or</u> with an amine of formula (V):

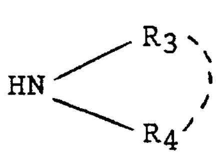

(V)

in which $R_3$ and $R_4$ have the previous meaning, to obtain a product of formula (IX):

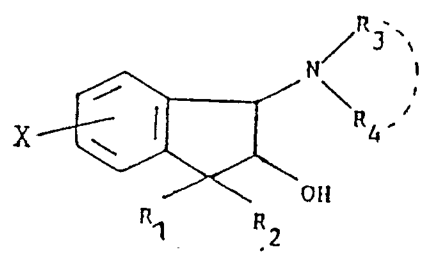

(IX)

of which the hydroxyl function is activated and which is treated with an amine of formula (X):

$NH_2R$ (X)

in which R has the previous meaning, to obtain a product of formula (XI):

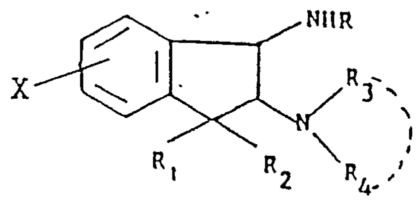

(XI)

which is condensed with an acid of formula (VIII) or a functional derivative of this acid:

Y-(Z)-COOH (VIII)

in which Z and Y have the previous meanings, to obtain a product of formula (I) in which A represents:

the group 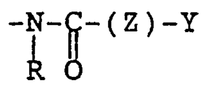 and B the group 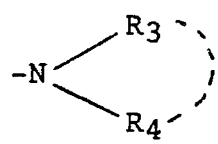

the said compounds of formula (I) being able to be resolved in order to obtain the optically active forms, and which are treated, if desired, with a mineral or organic acid, in order to obtain the salt.

2. Process according to claim 1, characterized in that an amine of formula (III) or (X) is used in which R represents a hydrogen atom, a methyl or ethyl radical, and an acid of formula (VIII) is used in which Z represents a $-CH_2-$ or

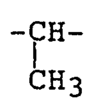

group and Y represents one of the following radicals: naphthyl, indenyl, benzo-[b]-thienyl, indolyl, benzofurannyl, quinolyl, benzimidazolyl, benzoxazolyl, benzothiazolyl, thienyl optionally substituted by a phenyl radical.

3. Process according to claim 1 or 2, characterized in that a product of formula (II), in which X, $R_1$ and $R_2$ represent a hydrogen atom, and an amine of formula (V) in which $R_3$ and $R_4$ form, together with the nitrogen atom to which they are linked, the pyrrolidine heterocycle, are used at the start.

4. Process according to any one of claims 1 to 3, characterized in that (trans) (±) N-[2,3-dihydro-2-(1-pyrrolidinyl)-1H-inden-1-yl]-N-methyl-4-benzo-[b]-thiophene acetamide and its salts with acids, and trans (±) N-[2,3-dihydro-2-(1-pyrrolidinyl)-1H-inden-1-yl]-N-methyl-1-naphthalene acetamide and its

addition salts with acids are prepared.

**5.** Preparation process for pharmaceutical compositions, characterized in that, as active ingredient, at least one of the products of formula (I) as defined in claim 1 or at least one of their addition salts with pharmaceutically acceptable acids is put in a form intended for this use.

**6.** As new industrial compounds, the products of formula (IV), (VI), (VII), (IX), (XI) as defined in claim 1, in which X does not represent a hydrogen atom.

## Ansprüche

**1.** Verbindungen der Formel (I)

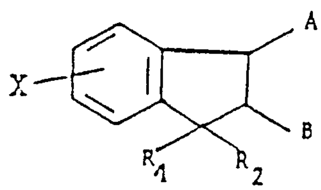

(I)

worin X ein Wasserstoffatom, ein Halogenatom, einen Alkylrest mit 1 bis 5 Kohlenstoffatomen, einen Alkoxyrest mit 1 bis 5 Kohlenstoffatomen bedeutet, $R_1$ und $R_2$, die identisch oder verschieden sind, ein Wasserstoffatom oder einen Alkylrest mit 1 bis 5 Kohlenstoffatomen bedeuten und A und B derart sind, daß einer der Substituenten A oder B ausgewählt ist aus:
- der Gruppe der Formel

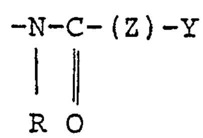

wobei R ein Wasserstoffatom oder ein Alkylrest mit 1 bis 5 Kohlenstoffatomen ist, Z eine lineare Alkylenkette $-(CH_2)_n$-ist, n eine ganze Zahl zwischen 0 und 5 ist oder eine verzweigte Alkylenkette mit 2 bis 8 Kohlenstoffatomen, Y bedeutet einen Naphthyl- oder Indenylrest oder einen heteromonocyclischen Rest mit 5 oder 6 Kettengliedern oder einen heterobicyclischen Rest, wobei die vorstehend erwähnten Reste gegebenenfalls substituiert sind durch einen oder mehrere Reste ausgewählt unter den Alkyl- oder Alkoxyresten mit 1 bis 5 Kohlenstoffatomen, Halogen, Hydroxyl, Trifluormethyl, Nitro, Amino, Monoalkyl- oder Dialkylamino oder Phenyl, das seinerseits gegebenenfalls substituiert ist durch einen oder mehrere Reste ausgewählt unter den Alkyl- oder Alkoxyresten mit 1 bis 4 Kohlenstoffatomen und Halogen, und der andere der Substituenten A und B ist ausgewählt aus
- der Gruppe der Formel

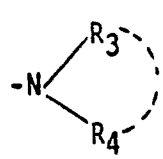

worin $R_3$ und $R_4$, die identisch oder verschieden sind, ein Wasserstoffatom oder einen Alkylrest mit 1 bis 5 Kohlenstoffatomen bedeuten, oder $R_3$ und $R_4$ bilden zusammen mit dem Stickstoffatom, an das sie gebunden sind, einen Heterozyklus mit 5 oder 6 Kettengliedern, der gegebenenfalls ein weiteres Heteroatom trägt, wie Sauerstoff, Stickstoff oder Schwefel, wobei diese Verbindungen der Formel (I) in allen möglichen enantiomeren und diastereoisomeren Formen und in Form der Additionssalze mit Säuren vorliegen können.

**2.** Verbindungen der Formel (I), wie sie in Anspruch 1 definiert sind, dadurch gekennzeichnet, daß die Gruppen A und B in der trans-Konfiguration sind, sowie ihre Additionssalze mit Säuren.

**3.** Verbindungen der Formel (I),wie sie in Anspruch 1 oder 2 definiert sind, dadurch gekennzeichnet, daß in der Gruppe

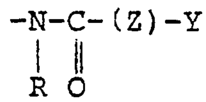

R ein Wasserstoffatom, einen Methyl- oder Ethylrest bedeutet, Z eine Gruppe

$-CH_2-$ oder 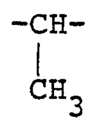

darstellt, Y einen Rest Naphthyl, Indenyl, Benzo-[b]-thienyl, Indolyl, Benzofuranyl, Chinolyl, Benzimidazolyl, Benzoxazolyl, Benzothiazolyl, Thienyl gegebenenfalls substituiert durch einen Phenylrest bedeutet, sowie ihre Additionssalze mit Säuren.

**4.** Verbindungen der Formel (I), wie sie in einem der Ansprüche 1 bis 3 definiert sind, dadurch gekennzeichnet, daß X, $R_1$ und $R_2$ eine Wasserstoffatom bedeuten und daß in der Gruppe

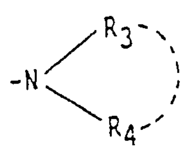

$R_3$ und $R_4$ mit dem Stickstoffatom, an das sie gebunden sind, den Heterozyklus Pyrrolidin bilden,sowie ihre Additionssalze mit Säuren.

**5.** (trans) (±) N-[2,3-Dihydro-2-(1-pyrrolidinyl-1H-inden-1-yl]-N-methyl-4-benzo-[b]-thiophenacetamid und seine Salze mit Säuren, und (trans) (±)N-[2,3-Dihydro-2-(1-pyrrolidinyl)-1H-inden-1-yl]-N-methyl-1-naphalinacetamid und seine Additionssalze mit Säuren.

**6.** Verfahren zur Herstellung der Produkte der Formel (I), deren Gruppen A und B in trans-Konfiguration sind, dadurch gekennzeichnet, daß man das Produkt der Formel (II)

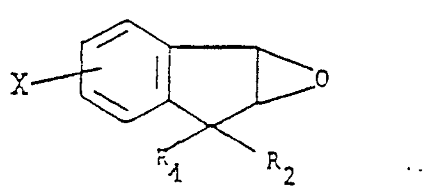

(II)

worin X,$R_1$ und $R_2$ die vorstehend gegebenen Bedeutungen haben, kondensiert
- entweder mit einem Amin der Formel (III)

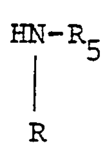

(III)

wobei R die vorstehend gegebene Bedeutung hat und $R_5$ eine Schutzgruppe der Aminfunktion und insbesondere eine Benzylgruppe darstellt, um ein Produkt der Formel (IV)

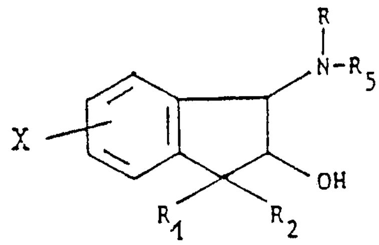

(IV)

zu erhalten, wovon man die Hydroxylfunktion aktiviert, das man mit einem Amin der Formel (V)

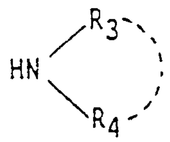

(V)

kondensiert, worin $R_3$ und $R_4$ die vorstehend angegebenen Bedeutungen haben, um eine Produkt der Formel (VI)

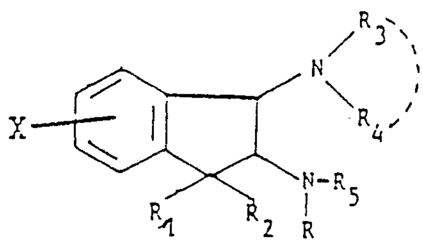

(VI)

zu erhalten, aus dem man die Schutzgruppe $R_5$ der Aminfunktion entfernt, um ein Produkt der Formel (VII)

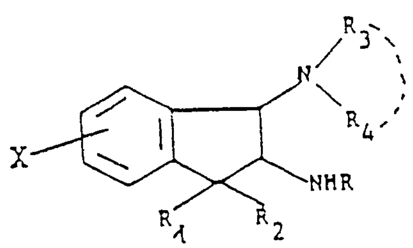

(VII)

zu erhalten, das man mit einer Säure der Formel (VIII) oder einem funktionellen Derivat dieser Säure

$$Y-(Z)-COOH \qquad (VIII)$$

behandelt, worin Y und Z die vorstehend angegebenen Definitionen haben, um eine Produkt der Formel (I) zu erhalten, worin

A die Gruppe 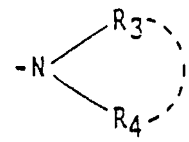 und

B die Gruppe 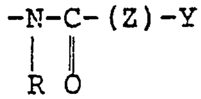

bedeuten,
- oder mit einem Amin der Formel (V)

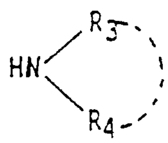

(V)

worin $R_3$ und $R_4$ die vorstehende Bedeutung haben, um eine Produkt der Formel (IX)

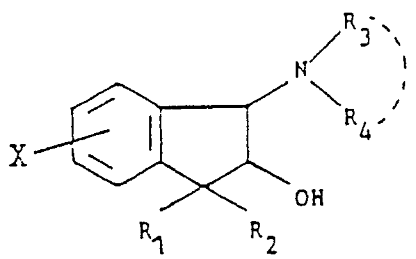

(IX)

zu erhalten,
von dem man die Hydroxylfunktion aktiviert und das man mit einem Amin der Formel (X)

$NH_2R$ (X)

behandelt, worin R die vorstehende Bedeutung hat, um ein Produkt der Formel (XI)

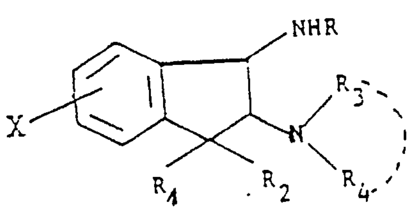

(XI)

zu erhalten, das man mit einer Säure der Formel (VIII) oder einem funktionellen Derivat dieser Säure

Y-(Z)-COOH (VIII)

kondensiert, worin Z und Y die vorstehenden Bedeutungen haben, um ein Produkt der Formel (I) zu erhalten, worin A die Gruppe

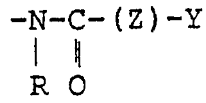 und B die Gruppe 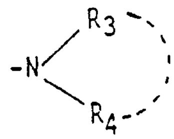

bedeuten, wobei diese Verbindungen der Formel (I) aufgetrennt werden können, um die optisch aktiven Formen zu erhalten, und daß man gewünschtenfalls mit einer Mineral- oder organischen Säure behandelt, um ein Salz zu erhalten.

7. Als Arzneimittel die Produkte der Formel (I), wie sie in einem der Ansprüche 1 - 4 definiert sind, sowie ihre Additionssalze mit pharmazeutisch annehmbaren Säuren.

8. Als Arzneimittel die Produkte gemäß Anspruch 5 sowie ihre Additionssalze mit pharmazeutisch annehmbaren Säuren.

9. Pharmazeutische Zusammensetzungen enthaltend als aktives Prinzip wenigstens eines der Arzneimittel, wie sie in den Ansprüchen 7 oder 8 definiert sind.

10. Als Zwischenprodukte die Produkte der Formeln (IV), (VI), (VII), (IX) und (XI), worin X nicht ein Wasserstoffatom bedeutet.

Patentansprüche für folgenden Vertragsstaat : ES

1. Verfahren zur Herstellung von Derivaten des Indans und ihren Additionssalzen mit Mineral- oder organischen Säuren der Formel (I)

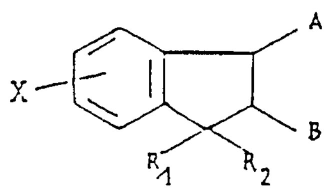

(I)

worin X ein Wasserstoffatom, ein Halogenatom, einen Alkylrest mit 1 bis 5 Kohlenstoffatomen, einen Alkoxyrest mit 1 bis 5 Kohlenstoffatomen bedeutet, $R_1$ und $R_2$, die identisch oder verschieden sind, ein Wasserstoffatom oder einen Alkylrest mit 1 bis 5 Kohlenstoffatomen bedeuten und A und B derart sind, daß einer der Substituenten A oder B ausgewählt ist aus:
- der Gruppe der Formel

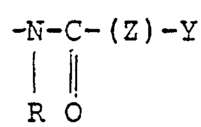

wobei R ein Wasserstoffatom oder ein Alkylrest mit 1 bis 5 Kohlenstoffatomen ist, Z eine lineare Alkylenkette $-(CH_2)_n$-ist, n eine ganze Zahl zwischen 0 und 5 oder eine verzweigte Alkylenkette mit 2 bis 8 Kohlenstoffatomen ist, Y einen Naphthyl- oder Indenylrest oder einen heteromonocyclischen Rest mit 5 oder 6 Kettengliedern oder heterobicyclischen Rest bedeutet, wobei die vorstehend erwähnten Reste gegebenenfalls substituiert sind durch einen oder zwei Reste ausgewählt unter den Alkyl- oder Alkoxyresten mit 1 bis 5 Kohlenstoffatomen, Halogen, Hydroxyl, Trifluormethyl, Nitro, Amino, Monoalkyl-oder Dialkylamino oder Phenyl, das seinerseits gegebenenfalls substituiert ist durch einen oder mehrere Reste ausgewählt unter den Alkyl- oder Alkoxyresten mit 1 bis 4 Kohlenstoffatomen und Halogen, und der andere der Substituenten A oder B ausgewählt ist aus
- der Gruppe der Formel

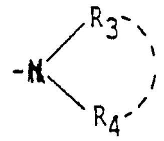

worin $R_3$ und $R_4$, die identisch oder verschieden sind, ein Wasserstoffatom oder einen Alkylrest mit 1 bis 5 Kohlenstoffatomen bedeuten, oder $R_3$ und $R_4$ bilden zusammen mit den Stickstoffatom, an das sie gebunden sind, einen Heterozyklus mit 5 oder 6 Kettengliedern, der gegebenenfalls ein weiteres Heteroatom trägt, wie Sauerstoff, Stickstoff oder Schwefel, wobei diese Verbindungen der Formel (I) in allen möglichen enantiomeren und diastereoisomeren Formen und unter der Form der Additionssalze mit Säuren vorliegen können,
dadurch gekennzeichnet, daß man das Produkt der Formel (II)

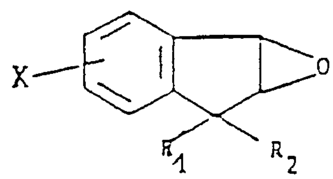

(II)

worin X, $R_1$ und $R_2$ die vorstehend angegebenen Bedeutungen haben, kondensiert
- entweder mit einem Amin der Formel (III)

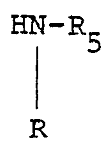

(III)

worin R die vorstehend angegebene Bedeutung hat und $R_5$ eine Schutzgruppe der Aminfunktion und insbesondere eine Benzylgruppe ist, um ein Produkt der Formel (IV)

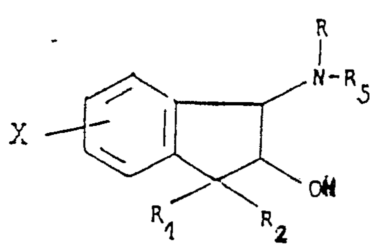

(IV)

zu erhalten, dessen Hydroxylfunktion man aktiviert und das man mit einem Amin der Formel (V)

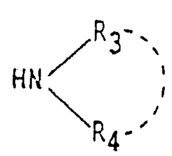

(V)

kondensiert, worin $R_3$ und $R_4$ die vorstehend angegebenen Bedeutungen haben, um ein Produkt der Formel (VI)

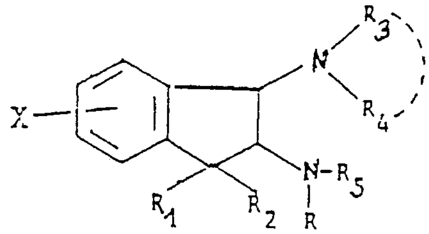

(VI)

zu erhalten, aus dem man die Schutzgruppe $R_5$ der Aminfunktion entfernt, um ein Produkt der Formel (VII)

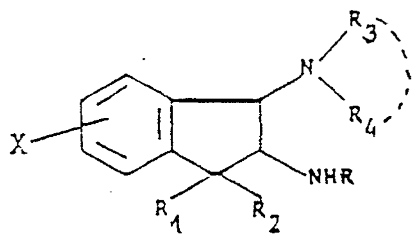

(VII)

zu erhalten, das man mit einer Säure der Formel (VIII) oder einem funktionellen Derivat dieser Säure

Y-(Z)-COOH (VIII)

behandelt, worin Y und Z die vorstehend angegebenen Bedeutungen haben, um ein Produkt der Formel (I) zu erhalten, worin

A die Gruppe 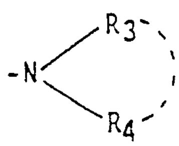 und B die Gruppe 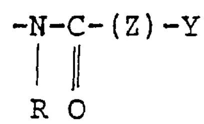

bedeutet,

- <u>oder</u> mit einem Amin der Formel (V)

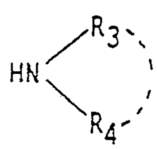

(V)

worin $R_3$ und $R_4$ die vorstehende Bedeutung haben, um ein Produkt der Formel (IX)

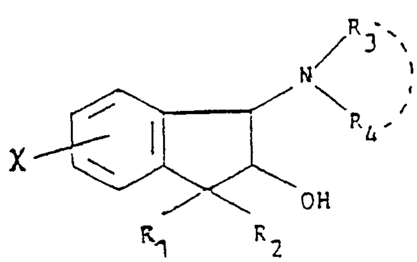

(IX)

zu erhalten, dessen Hydroxylfunktion man aktiviert und das man mit einem Amin der Formel (X)

$NH_2R$ (X)

behandelt, worin R die vorstehende Bedeutung hat, um ein Produkt der Formel (XI)

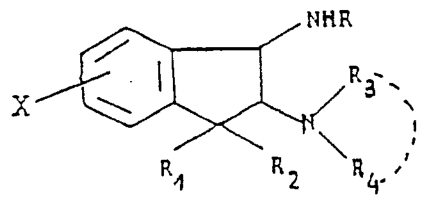

(XI)

zu erhalten, das man mit einer Säure der Formel (VIII) oder einem funktionellen Derivat dieser Säure

Y-(Z)-COOH (VIII)

kondensiert, worin Z und Y die vorstehenden Bedeutungen haben, um ein Produkt der Formel (I) zu erhalten,worin

A die Gruppe 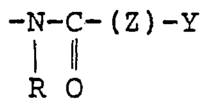 und B die Gruppe 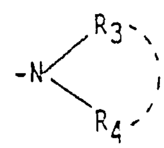

bedeuten,
wobei diese Verbindungen der Formel (I) aufgetrennt werden können, um die optisch aktiven Formen zu erhalten, und daß man gewünschtenfalls mit einer organischen oder Mineralsäure behandelt, um ein Salz zu erhalten.

2. Verfahren gemäß Anspruch 1, dadurch gekennzeichnet, daß man ein Amin der Formel (III) oder (X) verwendet, worin R ein Wasserstoffatom, einen Methyl- oder Ethylrest bedeutet, und eine Säure der Formel (VIII) verwendet, worin Z eine Gruppe

$-CH_2-$ oder 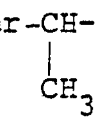

und Y einen Rest Naphthyl, Indenyl, Benzo-[b]-thienyl, Indolyl, Benzofuranyl, Chinolyl, Benzimidazolyl, Benzoxazolyl, Benzothiazolyl, Thienyl gegebenenfalls substituiert durch einen Phenylrest, bedeutet.

3. Verfahren gemäß Anspruch 1 oder 2, dadurch gekennzeichnet, daß man als Ausgangsprodukt ein Produkt der Formel (II) verwendet, worin X, $R_1$ und $R_2$ ein Wasserstoffatom bedeuten und ein Amin der Formel (V), worin $R_3$ und $R_4$ mit dem Stickstoffatom, an das sie gebunden sind, den Heterozyklus Pyrrolidin bildet.

Patentansprüche für folgenden Vertragsstaat : GR

1. Verfahren zur Herstellung von Derivaten des Indans und ihren Additionssalzen mit Mineral- oder organischen Säuren der Formel (I)

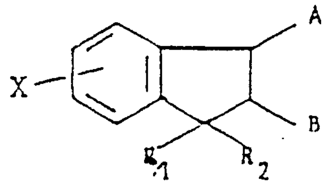

(I)

worin X ein Wasserstoffatom, ein Halogenatom, einen Alkylrest mit 1 bis 5 Kohlenstoffatomen, einen Alkoxyrest mit 1 bis 5 Kohlenstoffatomen bedeutet, $R_1$ und $R_2$, die identisch oder verschieden sind, ein Wasserstoffatom oder einen Alkylrest mit 1 bis 5 Kohlenstoffatomen bedeuten und A und B derart sind, daß einer der Substituenten A oder B ausgewählt ist aus:

- der Gruppe der Formel

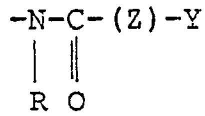

wobei R ein Wasserstoffatom oder ein Alkylrest mit 1 bis 5 Kohlenstoffatomen ist, Z eine lineare Alkylenkette $-(CH_2)_n$-ist, n eine ganze Zahl zwischen 0 und 5 oder eine verzweigte Alkylenkette mit 2 bis 8 Kohlenstoffatomen ist, Y einen Naphthyl- oder Indenylrest oder einen heteromonocyclischen Rest mit 5 oder 6 Kettengliedern oder heterobicyclischen Rest bedeutet, wobei die vorstehend erwähnten Reste gegebenenfalls substituiert sind durch einen oder zwei Reste ausgewählt unter den Alkyl- oder Akoxyresten mit 1 bis 5 Kohlenstoffatomen, Halogen, Hydroxyl, Trifluormethyl, Nitro, Amino, Monoalkyloder Dialkylamino oder Phenyl, das seinerseits gegebenenfalls substituiert ist durch einen oder mehrere Reste ausgewählt unter den Alkyl- oder Alkoxyresten mit 1 bis 4 Kohlenstoffatomen und Halogen, und der andere der Substituenten A oder B ausgewählt ist aus

- der Gruppe der Formel

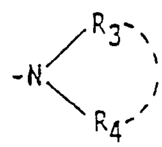

worin $R_3$ und $R_4$, die identisch oder verschieden sind, ein Wasserstoffatom oder einen Alkylrest mit 1 bis 5 Kohlenstoffatomen bedeuten, oder $R_3$ und $R_4$ bilden zusammen mit dem Stickstoffatom, an das sie gebunden sind, einen Heterozyklus mit 5 oder 6 Kettengliedern, der gegebenenfalls ein weiteres Heteroatom trägt, wie Sauerstoff, Stickstoff oder Schwefel, wobei diese Verbindungen der Formel (I) in allen möglichen enantiomeren und diastereoisomeren Formen und unter der Form der Addtionssalze mit Säuren vorliegen können,

dadurch gekennzeichnet, daß man das Produkt der Formel (II)

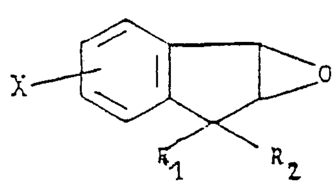

(II)

worin X, $R_1$ und $R_2$ die vorstehend angegebenen Bedeutungen haben, kondensiert

- entweder mit einem Amin der Formel (III)

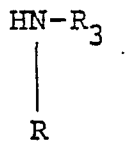

(III)

worin R die vorstehend angegebene Bedeutung hat und $R_5$ eine Schutzgruppe der Aminfunktion und insbesondere eine Benzylgruppe ist, um ein Produkt der Formel (IV)

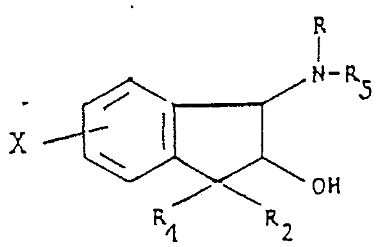

(IV)

zu erhalten, dessen Hydroxylfunktion man aktiviert und das man mit einem Amin der Formel (V)

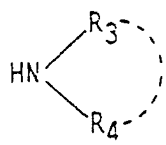

(V)

kondensiert, worin $R_3$ und $R_4$ die vorstehend angegebenen Bedeutungen haben, um ein Produkt der Formel (VI)

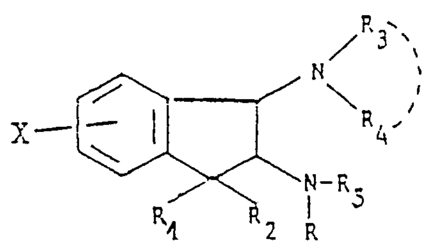

(VI)

zu erhalten, aus dem man die Schutzgruppe $R_5$ der Aminfunktion entfernt, um ein Produkt der Formel (VII)

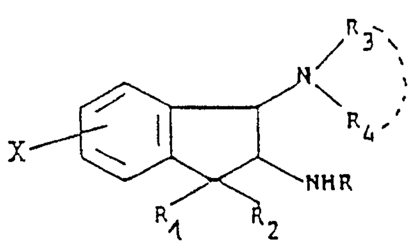

(VII)

zu erhalten, das man mit einer Säure der Formel (VIII) oder einem funktionellen Derivat dieser Säure

Y-(Z)-COOH (VIII)

behandelt, worin Y und Z die vorstehend angegebenen Bedeutungen haben, um ein Produkt der

Formel (I) zu erhalten, worin

A die Gruppe 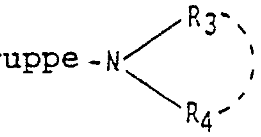 und B die Gruppe 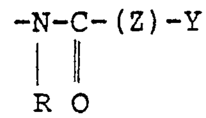

bedeutet,
- oder mit einem Amin der Formel (V)

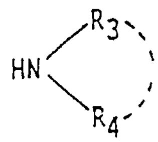

(V)

worin $R_3$ und $R_4$ die vorstehende Bedeutung haben, um ein Produkt der Formel (IX)

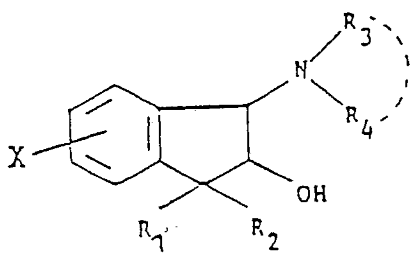

(IX)

zu erhalten, dessen Hydroxlfunktion man aktiviert und das man mit einem Amin der Formel (X)

$$NH_2R \qquad (X)$$

behandelt, worin R die vorstehende Bedeutung hat, um ein Produkt der Formel (XI)

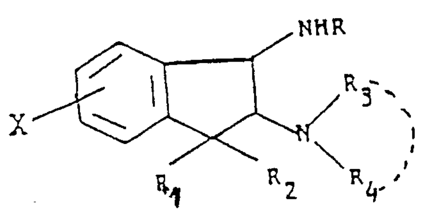

(XI)

zu erhalten, das man mit einer Säure der Formel (VIII) oder einem funktionellen Derivat dieser Säure

$$Y-(Z)-COOH \qquad (VIII)$$

kondensiert, worin Z und Y die vorstehenden Bedeutungen haben, um ein Produkt der Formel (I) zu erhalten, worin

A die Gruppe 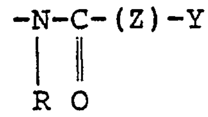 und B die Gruppe 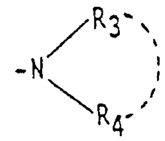

bedeuten,
wobei diese Verbindungen der Formel (I) aufgetrennt werden können, um die optisch aktiven Formen zu erhalten, und daß man gewünschtenfalls mit einer organischen oder Mineralsäure behandelt, um ein Salz zu erhalten.

2. Verfahren gemäß Anspruch 1, dadurch gekennzeichnet, daß man ein Amin der Formel (III) oder (X) verwendet, worin R ein Wasserstoffatom, einen Methyl- oder Ethylrest bedeutet, und eine Säure der Formel (VIII) verwendet,worin Z eine Gruppe

$-CH_2-$ oder 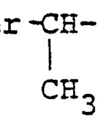

und Y einen Rest Naphthyl, Indenyl, Benzo-[b]-thienyl, Indolyl, Benzofuranyl, Chinolyl, Benzimidazolyl, Benzoxazolyl, Benzothiazolyl, Thienyl gegebenenfalls substituiert durch einen Phenylrest, bedeutet.

3. Verfahren gemäß Anspruch 1 oder 2, dadurch gekennzeichnet, daß man als Ausgangsprodukt ein Produkt der Formel (II) verwendet, worin X, $R_1$ und $R_2$ ein Wasserstoffatom bedeuten und ein Amin der Formel (V), worin $R_3$ und $R_4$ mit dem Stickstoffatom an das sie gebunden sind, den Heterozyklus Pyrrolidin bildet.

4. Verfahren gemäß einem der Ansprüche 1 bis 3, dadurch gekennzeichnet, daß man das (trans) (±) N-[2,3-Dihydro 2-(1-pyrrolidinyl-1H-inden 1-yl] N-methyl 4 benzo [b] tiophenacetamid und seine Salze mit Säuren,
und das trans (±) N-[2,3-Dihydro 2-(l-pyrrolidinyl) 1H-inden-l-yl] N-methyl 1 naphthalinacetamid und seine Additionssalze mit Säuren herstellt.

5. Verfahren zur Herstellung von pharmazeutischen Zusammensetzungen, dadurch gekennzeichnet, daß man als aktives Prinzip mindestens eines der Produkte der Formel (I), wie sie in Anspruch 1 definiert ist, oder mindestens eines seiner Säureadditionssalze mit pharmazeutisch annehmbaren Säuren unter einer für diesen Verwendungszweck bestimmten Form einsetzt.

6. Als neue industrielle Verbindungen die Produkte der Formel (IV), (VI), (VII), (IX), (XI), wie sie in Anspruch 1 definiert sind, worin X nicht ein Wasserstoffatom bedeutet.